# EUROPEAN PATENT APPLICATION

(11) **EP 3 718 531 A1**
(43) Date of publication of application: **07.10.2020**
(21) Application number: 18883530.0
(22) Date of filing: 30.11.2018
(51) Int. Cl.: A61K 9/00, A61K 39/395, C07K 16/24, C07K 16/28, A61K 47/18

(54) **LIQUID PREPARATION OF HUMANIZED ANTIBODY FOR TREATING IL-6-RELATED DISEASE**

(30) Priority: 30.11.2017 CN 201711239538
(71) Applicant: Bio-Thera Solutions, Ltd., Guangzhou, Guangdong 510530 (CN)
(72) Inventor: LIN, Jian, Guangzhou Guangdong 510530 (CN); LIU, Fan, Guangzhou Guangdong 510530 (CN); YUE, Rui, Guangzhou Guangdong 510530 (CN); WU, Zhihao, Guangzhou Guangdong 510530 (CN); WANG, Shengwu, Guangzhou Guangdong 510530 (CN); LI, Shengfeng, Guangzhou Guangdong 510530 (CN)
(74) Representative: Croce, Valeria
(86) International application number: PCT/CN2018/118503
(87) International publication number: WO 2019/105450

(57) **Abstract**

The present invention relates to a liquid preparation of a humanized antibody for treating an IL-6-related disease. The liquid preparation comprises 2-100 mg/mL of a recombinant humanized anti-human interleukin 6 receptor monoclonal antibody, 5-20 mM of a histidine salt buffer solution (or a buffer solution of a combination of 5-20 mM of a histidine salt and 5-20 mM of sodium acetate), 0.025-0.075% (volume ratio) of a surfactant, and 3-5% (mass-to-volume ratio) of a stabilizer and water for injection. The antibody preparation enhances the stability of the recombinant anti-human interleukin 6 receptor monoclonal antibody, prevents aggregation and degradation of the monoclonal antibody, and acid isomer increase. The preparation is applicable in stabilizing the structure and function of the humanized antibody.

## Description

### Technical Field

The invention relates to a liquid formulation of humanized antibody for treating interleukin 6 (IL-6) related diseases.

### Background Art

Humanized anti-interleukin 6 receptor antibodies are drugs useful for the treatment of rheumatoid arthritis (RA) [1] whose mechanisms are binding soluble and membrane-bound IL-6 receptors (sIL-6R and mIL-6R) and inhibiting sIL-6R and mIL-6R mediated signaling.

IL-6 plays a central role [2] in the pathogenic process of RA. IL-6 can activate proliferation of endothelial cells and generation of new blood vessels, expressing intercellular adhesion molecule-1 (ICAM-1) and vascular cell adhesion molecule-1 (VCAM-1) in vascular endothelial cells, and further promote the migration of lymphocytes, neutrophils and the like in blood into joints; it can activate osteoclasts to cause cartilage and bone damages; it can promote the differentiation of naive T cells to Th17, and Th17 in turn promotes the differentiation of T cells to Th1 type which aggravates the inflammatory response of RA and inhibits differentiation of Treg. An unbalanced Th17/Treg leads to weakened inhibiting effect and reduced immune tolerance of the immune system to inflammation, which is the major pathologic process of many autoimmune diseases and chronic inflammation. In addition, humanized anti-interleukin 6 receptor antibodies increase the availability of iron by reducing IL-6 stimulated production of hepcidin, thereby increasing hemoglobin level and improving anemia associated with RA; with JAK/STAT signaling of IL-6 inhibited, the levels of C-reactive protein (CRP) and serum amyloid A secreted by liver cells are rapidly reduced, the erythrocyte sedimentation rate is reduced, and the systemic inflammatory response is controlled. Humanized anti-interleukin-6 receptor antibodies can specifically block the binding of IL-6 and IL-6 receptor, reduce local inflammatory cell infiltration and inflammatory factor generation, reduce pains and swelling of joints, cartilage and bone joint damages caused by joint inflammation, reduce systemic symptoms such as fatigue, anorexia and anemia caused by inflammation, and has a good treatment effect for patients with moderate and severe rheumatoid arthritis where traditional anti-rheumatic chemical medicine treatments are ineffective.

RA cases can be found around the world. In recent years, most researchers consider that the incidence of RA is about 1%, and female patients take up a greater percent of about 3 times that of male patients. Rheumatic arthritis can occur in all ages, and adult patients thereof are mostly found in middle-aged women, especially during menopause. According to an American survey, the prevalence rate is 0.9% in the group aged from 35 to 44, and this number increases with age: 2.9% in the group aged from 55 to 64, and 4.90% in the group older than 65. [3] According to statistics, the prevalence rate of RA in China is about 0.24%-0.4% [4], and with the aging of the population, the prevalence rate of RA will also increase. RA is one of the major diseases causing labor loss and disability in China. Most RA conditions of patients are progressive and destructive. Within two years from onset of symptoms, 50% to 90% of patients had radiological changes in joint damages, about 50% of untreated patients become disabled within two years, 70% of untreated patients become disabled within three years, and once bone lesion occurs, it is irreversible [5]. Positive and correct treatment can relieve disease conditions in more than 80% of rheumatoid arthritis patients.

The antibody formulation developed by the invention comprises humanized anti-interleukin 6 receptor antibody, which is an active ingredient, and is used for treating relevant diseases caused by IL-6. In order to provide an antibody product with stable activity, it is necessary to develop a formulation that facilitates stable preservation of the antibody so that the function and structure of the antibody can be maintained for a long time.

### Summary of the Invention

It is an objective of the present invention to provide a stable liquid formulation comprising monoclonal antibodies.

The objective of the present invention is achieved by the following technical means:
In one aspect, the invention provides an antibody formulation, the antibody formulation comprising a humanized anti-interleukin 6 receptor antibody, a buffer system, a stabilizer and a surfactant. Specifically, the antibody formulation of present invention comprises the following ingredients:
(1) the antibody: 2-100 mg/mL humanized anti-IL-6 receptor antibody;
(2) the buffer system: the buffer system being formed in the formulation by a buffer, the buffer system being a buffer of 5-20 mM histidine salt, or a combination of 5-20 mM histidine salt and 5-20 mM sodium acetate;
(3) the surfactant: 0.1-1 g/L;
(4) the stabilizer: 30-400 mM;
(5) water for injection.

The pH of the antibody formulation is 5.0-7.0.

The humanized anti-interleukin 6 receptor antibody is expressed in CHO cells by genetic engineering methods and purified by a series of standard chromatographic steps. After the antibody is prepared, a pharmaceutical formulation is prepared.

As a preferred embodiment, the concentration of the humanized anti-IL-6 receptor antibody in the formulation is 10-90 mg/mL; as a more preferred embodiment, the concentration of the humanized anti-IL-6 receptor antibody in the formulation is 15-50 mg/mL; as a particularly preferred embodiment, the concentration of the humanized anti-IL-6 receptor antibody in the formulation is 18-25 mg/mL; as the most preferred embodiment, the concentration of the humanized anti-IL-6 receptor antibody in the formulation is 20 mg/mL.

As a preferred embodiment, the antibody comprises a heavy chain shown in SEQ ID NO. 1 and a light chain shown in SEQ ID NO. 2; as a more preferred embodiment, the antibody is BAT1806; further, BAT1806 comprises two heavy chains shown in SEQ ID NO. 1 and two light chains shown in SEQ ID NO. 2.

Wherein the stabilizer is selected from a combination of arginine hydrochloride and sucrose, or mannitol, or sodium chloride; further, the stabilizer is selected from the group consisting of 50-200 mM (10.533-42.132 g/L) arginine hydrochloride and 58-205 mM (20-70 g/L) sucrose; or the stabilizer is selected from 167-388 mM (30-70 g/L) mannitol; yet or the stabilizer is selected from 100-300 mM (5.85-17.55 g/L) sodium chloride.

Wherein the surfactant is one or more selected from polysorbate-20, polysorbate-80 and poloxamer 188. As a preferred embodiment, the surfactant is selected from polysorbate-80; further, the surfactant is selected from 0.1-0.7 g/L polysorbate-80.

As a preferred embodiment, the pH of the antibody formulation is 5.5-6.5; as a more preferred embodiment, the pH of the antibody formulation is between 6.0 and 6.4; as a still more preferred embodiment, the pH of the antibody formulation is 6.2.

As a preferred embodiment, the antibody formulation of the present invention comprises the following ingredients:
(1) 18-22 mg/mL humanized anti-IL-6 receptor antibody;
(2) 8-15 mM histidine salt buffer solution;
(3) 0.45 g/L to 0.65 g/L polysorbate-80;
(4) 40-60 mM arginine hydrochloride;
(5) 15-25 g/L sucrose;
(6) water for injection;
the pH is 6.0-6.4;
or preferably, it comprises the following ingredients:
(1) 18-22 mg/mL humanized anti-IL-6 receptor antibody;
(2) 8-15 mM histidine salt buffer solution;
(3) 0.45-0.65 g/L polysorbate-80;
(4) 30-45 g/L mannitol;
(5) water for injection;
(6) the pH is 6.0-6.4;
or yet preferably, it comprises the following ingredients:
(1) 18-22 mg/mL humanized anti-IL-6 receptor antibody;
(2) 8-15 mM histidine salt;
(3) 0.45-0.65 g/L polysorbate-80;
(4) 90-110 mM sodium chloride;
(5) water for injection;
the pH is 6.0-6.4.

As a more preferred embodiment, the antibody formulation of the present invention comprises the following ingredients:
(1) 20 mg/mL humanized anti-IL-6 receptor antibody;
(2) 10 mM histidine salt buffer;
(3) 0.5 g/L polysorbate-80;
(4) 50 mM arginine hydrochloride;
(5) 20 g/L sucrose;
(6) water for injection;
the pH is 6.2;
or more preferably, it comprises the following ingredients:
(1) 20 mg/mL humanized anti-IL-6 receptor antibody;
(2) 10 mM histidine salt buffer;
(3) 0.5 g/L polysorbate 80;
(4) 30 g/L mannitol;
(5) water for injection;
The pH is 6.2;
or more preferably, it comprises the following ingredients:
(1) 20 mg/mL humanized anti-IL-6 receptor antibody;
(2) 10 mM histidine salt buffer;
(3) 0.5 g/L polysorbate-80;
(4) 42 g/L mannitol;
(5) water for injection;
the pH is 6.2;
or more preferably, it comprises the following ingredients:
(1) 20 mg/mL humanized anti-IL-6 receptor antibody;
(2) 10 mM histidine buffer;
(3) 0.5 g/L polysorbate 80;
(4) 100 mM sodium chloride;
(5) water for injection;
the pH is 6.2.

The antibody formulation of the invention also comprises a base for adjusting the pH. In an exemplary embodiment of the present invention, the base is NaOH.

The antibody formulation is an aqueous formulation for injection. The formulation is suitable for subcutaneous injection or intravenous injection.

In another aspect, the invention also provides a method to prepare the antibody formulation, comprising the steps of:
(1) dissolving a weighed buffer, a stabilizer and a surfactant in water for injection;
(2) adjusting the liquid prepared in the step (1) with an aqueous sodium hydroxide until the pH is 5-7; preferably, the concentration of aqueous sodium hydroxide is 1 M;
(3) filtering the liquid prepared in the step (2) into an aseptic container; preferably, the pore size of the filter membrane being 0.22 um for filtering bacteria and fungi; and
(4) adding the liquid prepared in the step (3) into an antibody solution.

The antibody formulation is a pharmaceutical formulation for treating IL-6 related diseases; specifically, the IL-6 related diseases include: adult rheumatoid arthritis, systemic juvenile idiopathic arthritis, polyarticular juvenile idiopathic arthritis, giant cell arteritis, giant lymph node hyperplasia, cytokine storm caused by immunotherapy, adult Still's disease, recurrent polychondritis, type II diabetes, ankylosing spondylitis, thyroid-associated ocular diseases, cardiovascular diseases caused by rheumatoid arthritis, polymyalgia rheumatica, acute graft-versus-host disease, non-ST-segment elevation myocardial infarction, systemic lupus erythematosus, schizophrenia, uveitis, ovarian cancer, anti-neutrophil cytoplasmic antibody-associated vasculitis, neuromyelitis optica, chronic glomerulonephritis, colorectal cancer and the like; as a preferred embodiment, the IL-6 related diseases include: adult rheumatoid arthritis, systemic juvenile idiopathic arthritis, polyarticular juvenile idiopathic arthritis, giant cell arteritis, and giant lymph node hyperplasia.

In a preferred embodiment of the invention, the antibody is BAT1806, a human monoclonal antibody which is produced in CHO-K1 using recombinant DNA techniques, settled to obtain a culture supernatant, and purified. The mechanism of BAT1806 is to specifically bind soluble and membrane-bound IL-6 receptors (sIL-6R and mIL-6R), and inhibit sIL-6R and mIL-6R mediated signaling. BAT1806 has a good treatment effect on IL-6 related diseases such as adult rheumatoid arthritis, systemic juvenile idiopathic arthritis, polyarticular juvenile idiopathic arthritis, giant cell arteritis, giant lymph node hyperplasia and cytokine storm caused by immunotherapy.

In the antibody formulations provided by the invention, in order to keep the stability of the humanized anti-interleukin 6 receptor antibody, an appropriate buffer system was selected, a stabilizer was optimized and a surfactant was added, and through a large amount of research work, antibody formulations have been developed to remarkably inhibit the formation of acidic peaks, dimers, aggregates, degradants and insoluble microparticles during freezing/thawing cycles, long-term storage and temperature variation processes. In particular, the humanized anti-interleukin 6 receptor antibodies remain stable in the above formulations after at least 5 freeze-thaw cycles, stable at room temperature for at least 6 months, and stable at 4 °C for 36 months. Therefore, the antibody formulations of the present invention can be used for stably storing the humanized anti-interleukin 6 receptor antibody for clinical treatment, which has a great significance for treating relevant diseases caused by IL-6.

### Brief Description of the Drawings

Figure 1A: IEC-HPLC main peak analysis of pH selection (40 °C high temperature) for the humanized anti-interleukin 6 receptor antibody BAT1806 formulation;
Figure 1B: SEC-HPLC main peak analysis of pH selection (40 °C high temperature) for the humanized anti-interleukin 6 receptor antibody BAT1806 formulation.
Figure 2A: SEC main peak analysis of stabilizer selection (50 °C high temperature) for the humanized anti-interleukin 6 receptor antibody BAT1806 formulation;
Figure 2B: SEC aggregate analysis of stabilizer selection (50 °C high temperature) for the humanized anti-interleukin 6 receptor antibody BAT1806 formulation;
Figure 2C: SEC fragment analysis of stabilizer selection (50 °C high temperature) for the humanized anti-interleukin 6 receptor antibody BAT1806 formulation;
Figure 2D: IEC main peak analysis of stabilizer selection (50 °C high temperature) for the humanized anti-interleukin 6 receptor antibody BAT1806 formulation;
Figure 2E: IEC acidic peak analysis of stabilizer selection (50 °C high temperature) for the humanized anti-interleukin 6 receptor antibody BAT1806 formulation;
   Illustrations (Figures 2A-2E):
   ZT (PB): 15 mM PBS buffer, 5% sucrose, protein concentration 20 mg/mL, pH = 6.5;
   ZT (His): 10 mM His buffer, 5% sucrose, protein concentration 20 mg/mL, pH = 6.2;
   Arg-HCL: 10 mM His buffer, 100 mM arginine hydrochloride, protein concentration 20 mg/mL, pH = 6.2;
   Pro: 10 mM His buffer, 100 mM proline, protein concentration 20 mg/mL, pH = 6.2;
   NaCl: 10 mM His buffer, 100 mM NaCl, protein concentration 20 mg/mL, pH = 6.2;
   GLC: 10 mM His buffer, 4.2% mannitol, protein concentration 20 mg/mL, pH = 6.2;
   Arg-HCl + ZT: 10 mM His buffer, 50 mM arginine hydrochloride, 2% sucrose, protein concentration 20 mg/mL; pH = 6.2;
   Arg-HCl (NaAC): 10 mM sodium acetate buffer, 100 mM arginine hydrochloride, protein concentration 20 mg/mL, pH = 6.2;
   0d: Day 0; 4d: Day 4; 8d: Day 8; 12d: Day 12.

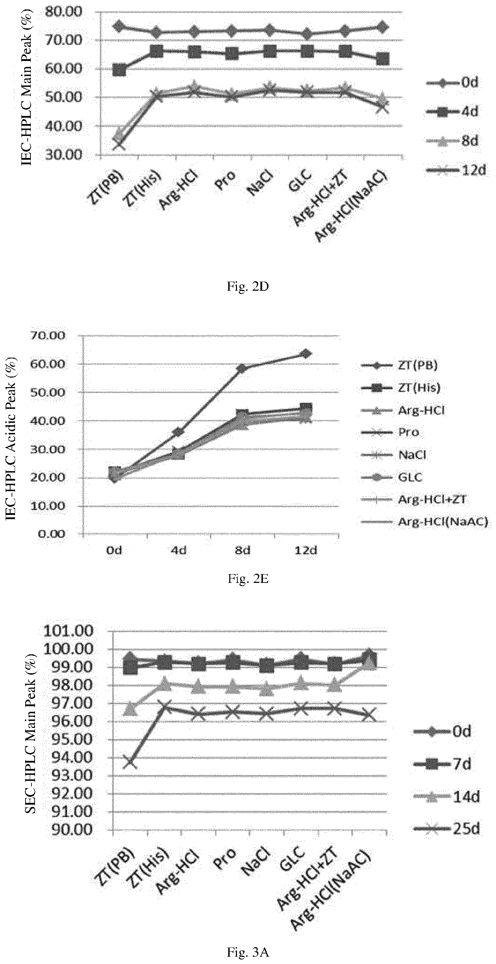

Figure 3A: SEC main peak analysis of stabilizer selection (40 °C high temperature) for the humanized anti-interleukin 6 receptor antibody BAT1806 formulation;

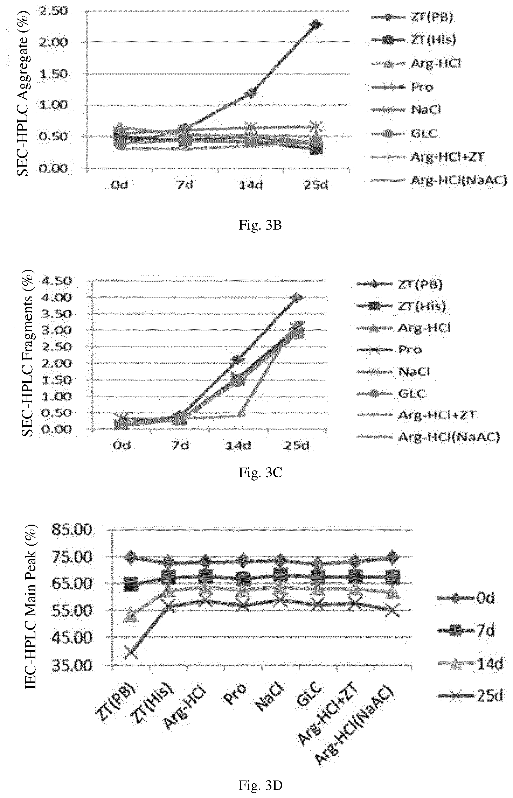

Figure 3B: SEC aggregate analysis of stabilizer selection (40 °C high temperature) for the humanized anti-interleukin 6 receptor antibody BAT1806 formulation;
Figure 3C: SEC fragment analysis of stabilizer selection (40 °C high temperature) for the humanized anti-interleukin 6 receptor antibody BAT1806 formulation;
Figure 3D: IEC main peak analysis of stabilizer selection (40 °C high temperature) for the humanized anti-interleukin 6 receptor antibody BAT1806 formulation;

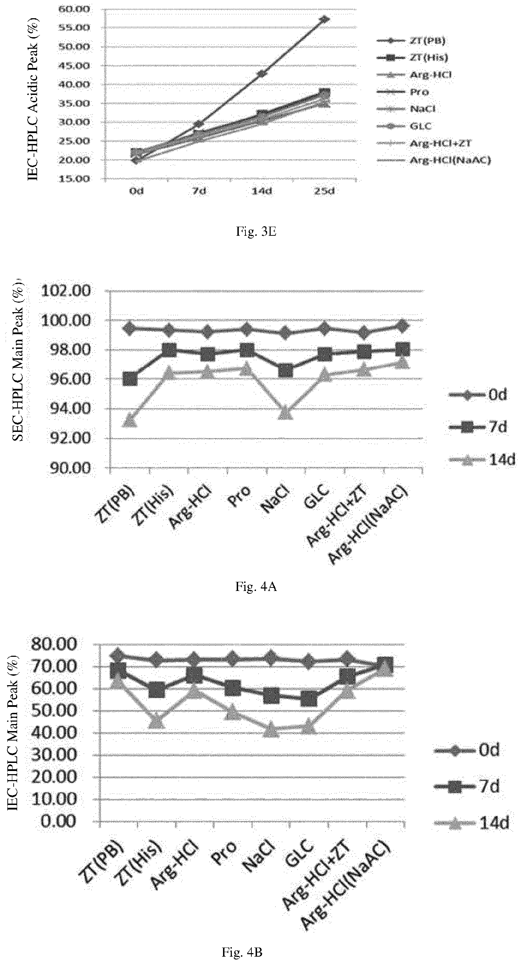

Figure 3E: IEC acidic peak analysis of stabilizers selection (40 °C high temperature) for the humanized anti-interleukin 6 receptor antibody BAT1806 formulation;
   Illustrations (Figures 3A-3E):
   ZT (PB): 15 mM PBS buffer, 5% sucrose, protein concentration 20 mg/mL, pH = 6.5;
   ZT (His): 10 mM His buffer, 5% sucrose, protein concentration 20 mg/mL, pH = 6.2;
   Arg-HCL: 10 mM His buffer, 100 mM arginine hydrochloride, protein concentration 20 mg/mL, pH = 6.2;
   Pro: 10 mM His buffer, 100 mM proline, protein concentration 20 mg/mL, pH = 6.2;
   NaCl: 10 mM His buffer, 100 mM NaCl, protein concentration 20 mg/mL, pH = 6.2;
   GLC: 10 mM His buffer, 4.2% mannitol, protein concentration 20 mg/mL, pH = 6.2;
   Arg-HCl + ZT: 10 mM His buffer, 50 mM arginine hydrochloride, 5% sucrose, protein concentration 20 mg/mL; pH = 6.2;
   Arg-HCl (NaAC): 10 mM sodium acetate buffer, 100 mM arginine hydrochloride, protein concentration 20 mg/mL, pH = 6.2;
   0d: Day 0; 7d: Day 7; 14d: Day 14; 25d: Day 25.
Figure 4A: SEC main peak analysis of stabilizer selection (light 4000 Lx) for the humanized anti-interleukin 6 receptor antibody BAT1806 formulation;
Figure 4B: IEC main peak analysis of stabilizer selection (light 4000 Lx) for the humanized anti-interleukin 6 receptor antibody BAT1806 formulation;
   Illustrations (Figures 4A-4B):
   ZT (PB): 15 mM PBS buffer, 5% sucrose, protein concentration 20 mg/mL, pH = 6.5;
   ZT (His): 10 mM His buffer, 5% sucrose, protein concentration 20 mg/mL, pH = 6.2;
   Arg-HCL: 10 mM His buffer, 100 mM arginine hydrochloride, protein concentration 20 mg/mL, pH = 6.2;
   Pro: 10 mM His buffer, 100 mM proline, protein concentration 20 mg/mL, pH = 6.2;
   NaCl: 10 mM His buffer, 100 mM NaCl, protein concentration 20 mg/mL, pH = 6.2;
   GLC: 10 mM His buffer, 4.2% mannitol, protein concentration 20 mg/mL, pH = 6.2;
   Arg-HCl + ZT: 10 mM His buffer, 50 mM arginine hydrochloride, 5% sucrose, protein concentration 20 mg/mL; pH = 6.2;
   Arg-HCl (NaAC): 10 mM sodium acetate buffer, 100 mM arginine hydrochloride, protein concentration 20 mg/mL, pH = 6.2;
   0d: Day 0; 7d: Day 7; 14d: Day 14.

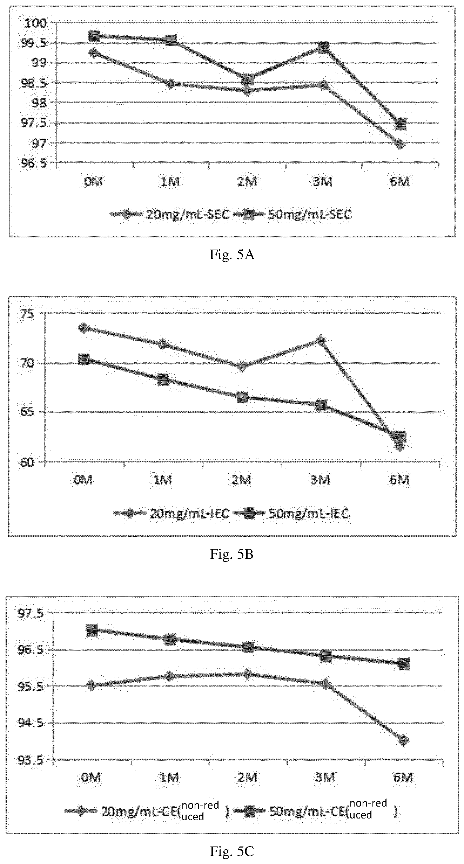

Figure 5A is a graph of SEC trends for Formulations E and F under acceleration conditions;
Figure 5B is a graph of IEC trends for Formulations E and F under acceleration conditions;
Figure 5C is a graph of CE (non-reduced) trends for Formulations E and F under acceleration conditions.

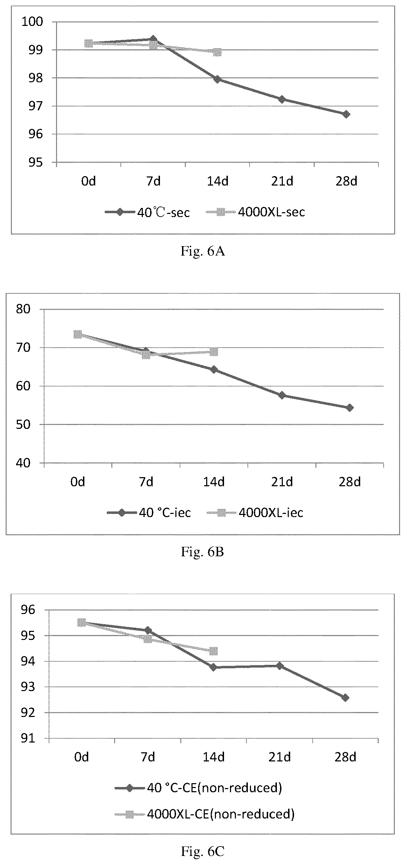

Figure 6A is a graph of SEC trends for Formulation E under high temperature and light;
Figure 6B is a graph of IEC trends for Formulation E under high temperature and light;
Figure 6C is a graph of CE (non-reduced) trends for Formulation E under conditions of high temperature and light.

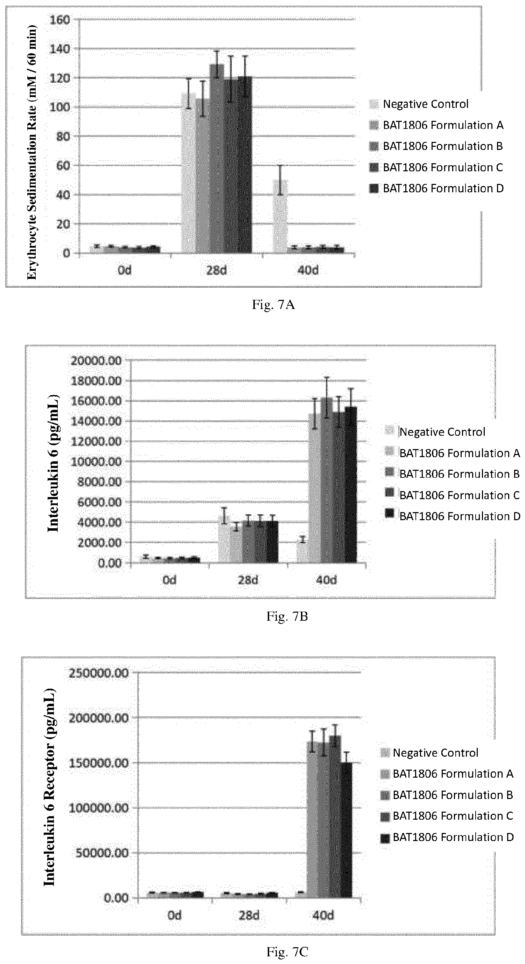

Figure 7A: evaluation of the therapeutic effect of the humanized anti-interleukin 6 receptor antibody BAT1806 in a CIA model: ESR: erythrocyte sedimentation rate; *P<0.05
Figure 7B: evaluation of the therapeutic effect of the humanized anti-interleukin 6 receptor antibody BAT1806 in the CIA model: IL-6: interleukin 6; *P<0.05
Figure 7C: evaluation of the therapeutic effect of the humanized anti-interleukin 6 receptor antibody BAT1806 in the CIA model; IL-6R: interleukin 6 receptor; *P<0.05
   Illustrations (Figures 7A-7C):
   0d: before modeling; 28d: after successful modeling/before dosing; 40d: Day 7 post-dosing.

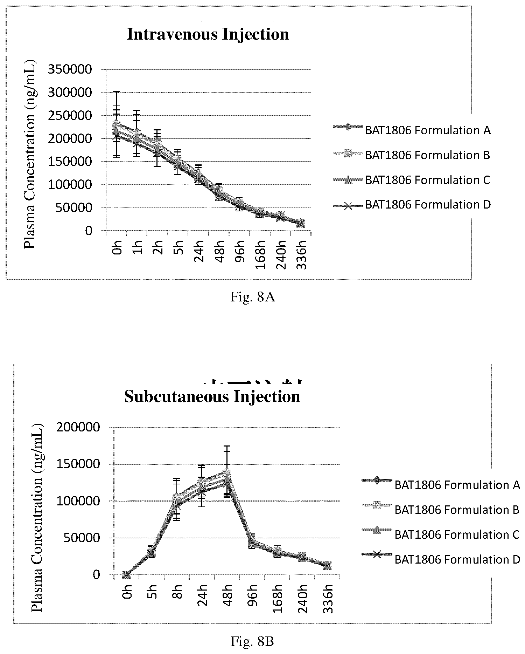

Figure 8A: intravenous injection pharmacokinetic study of the humanized anti-interleukin 6 receptor antibody BAT1806;
Figure 8B: subcutaneous injection pharmacokinetic study of the humanized anti-interleukin 6 receptor antibody BAT1806.

### Detailed Description of the Invention

The present invention provides antibody formulation formulas by selecting buffer solutions and stabilizers, to enhance the stability of the humanized anti-interleukin 6 receptor antibody formulation, and prevent monoclonal antibody aggregation, degradation and acidic isomer increase.

Terms as "stability" and "stable" used herein refer to the situation that in a liquid formulation comprising an antibody (including antibody fragments thereof), the antibody (including antibody fragments thereof) does not, or only rarely, aggregate, degrade, or fragment under given production, formulation, transportation, and/or storage conditions. "Stable" formulation maintains biological activity under given production, formulation, transportation and/or storage conditions. The stability of the antibody, including antibody fragments thereof, can be assessed by measuring the degree of aggregation, degradation or fragmentation and the like of the formulation by techniques such as SEC-HPLC, IEC-HPLC, CE-SDS, etc.

It should be noted that in the present invention, where a buffer or buffer system is included in a formulation, it is also meant that the buffer is included in the formulation and the buffer system is formed in the formulation by the buffer.

In one embodiment of the invention, the concentration of the monoclonal antibody in the antibody formulation is about 2-100 mg/mL; as a preferred embodiment, the concentration of the monoclonal antibody in the antibody formulation is 10-90 mg/mL; as a more preferred embodiment, the concentration of the monoclonal antibody in the antibody formulation is 15-50 mg/mL; as a yet more preferred embodiment, the concentration of the monoclonal antibody in the antibody formulation is 18-25 mg/mL; as a particularly preferred embodiment, the concentration of the monoclonal antibody in the antibody formulation is 18-22 mg/mL; as the most preferred embodiment, the concentration of the monoclonal antibody in the antibody formulation is 20 mg/mL. In this embodiment, the effects of a total of six buffer systems such as phosphate (PB) buffer, histidine (His) buffer, citrate CB (NMS) buffer, phosphate + acetic acid (HAC) buffer, citrate + acetic acid buffer, histidine + sodium acetate (NaAC) buffer on antibody stability were evaluated. Among these buffers, histidine buffer and histidine + sodium acetate buffer had the best effect, followed by citrate buffer and phosphate + acetic buffer, and PB buffer alone had the worst effect.

In another embodiment of the invention, the concentration of the monoclonal antibody in the antibody formulation is about 2-100 mg/mL; as a preferred embodiment, the concentration of the monoclonal antibody in the antibody formulation is 10-90 mg/mL; as a more preferred embodiment, the concentration of the monoclonal antibody in the antibody formulation is 15-50 mg/mL; as a yet more preferred embodiment, the concentration of the monoclonal antibody in the antibody formulation is 18-25 mg/mL; as a particularly preferred embodiment, the concentration of the monoclonal antibody in the antibody formulation is 18-22 mg/mL; as the most preferred embodiment, the concentration of the monoclonal antibody in the antibody formulation is 20 mg/mL. In this embodiment, the effect of adding appropriate stabilizers such as sucrose (ZT), mannitol (GLC), arginine hydrochloride (Arg-Hcl), proline (Pro), sodium chloride (NaCl) to the buffer on antibody stability was evaluated. The addition of the stabilizers further increases the stability of the formulation, with different types of stabilizers having no significant differences in their effects on antibody stability.

In another embodiment of the invention, the concentration of the monoclonal antibody in the antibody formulation is about 2-100 mg/mL; as a preferred embodiment, the concentration of the monoclonal antibody in the antibody formulation is 10-90 mg/mL; as a more preferred embodiment, the concentration of the monoclonal antibody in the antibody formulation is 15-50 mg/mL; as a yet more preferred embodiment, the concentration of the monoclonal antibody in the antibody formulation is 18-25 mg/mL; as a particularly preferred embodiment, the concentration of the monoclonal antibody in the antibody formulation is 18-22 mg/mL; as the most preferred embodiment, the concentration of the monoclonal antibody in the antibody formulation is 20 mg/mL. In this embodiment, the effect of the addition of appropriate surfactants (detergents) such as polysorbate-20, polysorbate-80, and poloxamer 188 to the formulation containing appropriate buffers and stabilizers on insoluble microparticles in the formulation after repeated freeze-thaw cycles was evaluated. Different types of the foregoing surfactants can reduce the generation of insoluble particles in the formulation after freeze-thaws, with different types of surfactants having no significant differences in their effects.

Preferred formulations were selected by selecting buffers, stabilizers and surfactants. One of the formulas may be as follows: 20 mg/mL effective amount of humanized anti-interleukin 6 receptor antibody, 10 mM histidine salt buffer, 0.5 g/L polysorbate-80, 50 mM arginine hydrochloride, 20 g/L sucrose, water for injection, pH 6.0-6.4; another formula may be as follows: 20 mg/mL effective amount of humanized anti-IL-6 receptor antibody, 10 mM histidine salt buffer, 0.5 g/L polysorbate-80, 30 g/L mannitol, water for injection, pH 6.0-6.4; another formula may be as follows: 20 mg/mL effective amount of humanized anti-IL-6 receptor antibody, 10 mM histidine salt buffer, 0.5 g/L polysorbate-80, 42 g/L mannitol, water for injection, pH 6.0-6.4; another formula may also be as follows: 20 mg/mL effective amount of humanized anti-IL-6 receptor antibody, 10 mM histidine hydrochloride, 0.5 g/L polysorbate-80, 100 mM sodium chloride, water for injection, pH 6.0-6.4; another formula may be as follows: 50 mg/mL effective amount of humanized anti-interleukin 6 receptor antibody, 10 mM histidine salt buffer, 0.5 g/L polysorbate-80, 50 mM arginine hydrochloride, 20 g/L sucrose, water for injection, pH 6.0-6.4.

Among the various ingredients of the humanized anti-IL-6 receptor antibody formulations of the present invention, the buffer system functions as one of the most critical control links, and in order to ensure good stability, the buffer system is preferably a histidine salt buffer system. In addition to providing an optimal pH range for the antibody, the buffer may also function as a stabilizer, and it is necessary to select the types of the buffer. The extents to which the stabilities of different antibody types are affected by different factors are different, and so are the results. The design of the formulation system of the present invention is several candidates that the inventors have designed based on extensive experiences. However, the final experimental results were still unpredictable or required long-term storage testing. The exogenous buffer system does not have an important effect on the stability of all types of antibody formulations, for example, for another antibody such as adalimumab, according to what is known about stability selecting results, the most critical factor is the choice of stabilizer, rather than the buffer system, because the buffering effect of the high-concentration protein itself can maintain the pH within the optimal range during the storage period.

According to the evaluation of the stability, *in vitro* and *in vivo* efficacy, pharmacokinetics of the above preferred formulations, the antibody formulations of the present invention can remain stable for at least 6 months at room temperature, 36 months at 4 °C, and remain stable after at least 5 freeze-thaw cycles. The preferred formulations described above have similar *in vivo* and *in vitro* pharmacodynamic and pharmacokinetic characteristics.

The liquid formulations containing the monoclonal antibody provided by the invention provide formulation combinations capable of stably storing the active ingredients, and the formulation formulas of the preferable formulations are as follows, respectively shown as Formulations A, B, C, D, E and F:

**Table 1A. Ingredient List for Humanized Anti-Interleukin 6 Receptor Antibody BAT1806 Formulation A**

| Name of Ingredient | Amount in Formula | Content in 4 mL Materials | Function |
|---|---|---|---|
| Humanized anti-interleukin 6 receptor antibody BAT1806 | 20 mg/mL | 80 mg | Active substance |
| Histidine | 1.008 g/L | 0.004032 g | Buffer |
| Histidine hydrochloride salt | 0.734 g/L | 0.002936 g | Buffer |
| Arginine hydrochloride salt | 10.533 g/L | 0.042132 g | Stabilizer |
| Sucrose | 20 g/L | 0.08 g | Stabilizer |
| Polysorbate-80 | 0.5 g/L (0.05%) | 0.002 g | Detergent |

**Table 1B. Ingredient List for Humanized Anti-Interleukin 6 Receptor Antibody BAT1806 Formulation B**

| Name of Ingredient | Amount in Formula | Content in 4 mL Materials | Function |
|---|---|---|---|
| Humanized anti-interleukin 6 receptor antibody BAT1806 | 20 mg/mL | 80 mg | Active substance |
| Histidine | 1.008 g/L | 0.004032 g | Buffer |
| Histidine hydrochloride salt | 0.734 g/L | 0.002936 g | Buffer |
| Mannitol | 30 g/L(3%) | 0.120 g | Stabilizer |
| Polysorbate-80 | 0.5 g/L(0.05%) | 0.002 g | Detergent |

**Table 1C. Ingredient List for Humanized Anti-Interleukin 6 Receptor Antibody BAT1806 Formulation C**

| Name of Ingredient | Amount in Formula | Content in 4 mL Materials | Function |
|---|---|---|---|
| Humanized anti-interleukin 6 receptor antibody BAT1806 | 20 mg/mL | 80 mg | Active substance |
| Histidine | 1.008 g/L | 0.004032 g | Buffer |
| Histidine hydrochloride salt | 0.734 g/L | 0.002936 g | Buffer |
| Mannitol | 42 g/L (4.2%) | 0.168 g | Stabilizer |
| Polysorbate-80 | 0.5 g/L (0.05%) | 0.002 g | Detergent |

**Table 1D. Ingredient List for Humanized Anti-Interleukin 6 Receptor Antibody BAT1806 Formulation D**

| Name of Ingredient | Amount in Formula | Content in 4 mL Materials | Function |
|---|---|---|---|
| Humanized anti-interleukin 6 receptor antibody BAT1806 | 20 mg/mL | 80 mg | Active substance |
| Histidine | 1.008 g/L | 0.004032 g | Buffer |
| Histidine hydrochloride salt | 0.734 g/L | 0.002936 g | Buffer |
| Sodium chloride | 5.85 g/L | 0.0234 g | Stabilizer |
| Polysorbate-80 | 0.5 g/L (0.05%) | 0.002 g | Detergent |

**Table 1E. Ingredient List for Humanized Anti-Interleukin 6 Receptor Antibody BAT1806 Formulation E**

| Name of Ingredient | Amount in Formula | Content in 4 mL Materials | Function |
|---|---|---|---|
| Humanized anti-interleukin 6 receptor antibody BAT1806 | 20 mg/mL | 80 mg | Active substance |
| Histidine | 0.81 g/L | 0.00324 g | Buffer |
| Histidine hydrochloride salt | 1.01 g/L | 0.00404 g | Buffer |
| Arginine hydrochloride salt | 10.533 g/L | 0.042132 g | Stabilizer |
| Sucrose | 20 g/L | 0.08 g | Stabilizer |
| Polysorbate-80 | 0.5 g/L (0.05%) | 0.002 g | Detergent |

**Table 1F. Ingredient List for Humanized Anti-Interleukin 6 Receptor Antibody BAT1806 Formulation F**

| Name of Ingredient | Amount in Formula | Content in 4 mL Materials | Function |
|---|---|---|---|
| Humanized anti-interleukin 6 receptor antibody BAT1806 | 50 mg/mL | 200 mg | Active substance |
| Histidine | 0.81 g/L | 0.00324 g | Buffer |
| Histidine hydrochloride salt | 1.01 g/L | 0.00404 g | Buffer |
| Arginine hydrochloride salt | 10.533 g/L | 0.042132 g | Stabilizer |
| Sucrose | 20 g/L | 0.08 g | Stabilizer |
| Polysorbate-80 | 0.5 g/L (0.05%) | 0.002 g | Detergent |

In summary, the present invention provides a formulation formula of an antibody for protecting monoclonal antibodies, which may comprise 2-100 mg/mL antibody, 5-20 mM histidine salt buffer, 0.25-1g/L polysorbate-80, and a stabilizer selected from the group of 50-120 mM arginine hydrochloride in combination with 10-50g/L sucrose, or 10-50g/L mannitol, or 50-120 mM sodium chloride, with a pH of 5.0-7.0. A preferred formula is as follows: 20 mg/mL antibody, 10 mM histidine salt buffer, 0.5g/L polysorbate-80, 50 mM arginine hydrochloride, 20g/L sucrose, pH adjusting sodium hydroxide and water for injection, pH 5.0-7.0 (abbreviated as Formulation A). Another preferred formula is as follows: 20 mg/mL antibody, 10 mM histidine salt buffer, 0.5g/L polysorbate-80, 30g/L mannitol, pH adjusting sodium hydroxide and water for injection, pH 5.0-7.0 (abbreviated as Formulation B). Another preferred formula is as follows: 20 mg/mL antibody, 10 mM histidine salt buffer, 0.5g/L polysorbate-80, 42g/L mannitol, pH adjusting sodium hydroxide and water for injection, pH 5.0-7.0 (abbreviated as Formulation C). Another preferred formula may also be as follows: 20 mg/mL antibody, 10 mM histidine salt, 0.5g/L polysorbate-80, 100 mM sodium chloride, pH adjusting sodium hydroxide and water for injection, pH 5.0-7.0 (abbreviated as Formulation D). Another formula may be as follows: 50 mg/mL antibody, 10 mM histidine salt buffer, 0.5g/L polysorbate-80, 50 mM arginine hydrochloride, 20g/L sucrose, pH adjusting sodium hydroxide and water for injection, pH 6.0-6.4 (abbreviated as Formulation F). In summary, in the antibody formulation provided by the invention, by selecting an appropriate buffer system, optimizing a stabilizer and adding a surfactant, the increase of acidic peaks, aggregates, degradants and insoluble particles in a freezing/thawing cycle, long-term storage and temperature and light change process can be effectively inhibited, and the active ingredients can be stably stored for a long time. Wherein the selection of buffer system plays the most important role for the stabilization of formulation, and compared with phosphate buffer, histidine salt buffer can significantly inhibit the formation of acidic isomers, aggregates and degradants in long-term storage and temperature change process.

The technical solution of the present invention is further illustrated by the following specific examples, which are not intended to limit the scope of the present invention. Other non-essential modifications and adaptations of the invention according to the inventive concept are within the scope of the invention.

It should be noted that in the present invention, the "mass to volume ratio" is the ratio of the mass of the ingredients to the volume of the formulation.

A "histidine salt buffer system" is a combination of histidine and histidine hydrochloride. As a preferred embodiment, a buffer containing 0.81 g/L histidine and 1.01 g/L histidine hydrochloride is preferred.

### Antibody Sample

The BAT1806 antibody is a humanized anti-interleukin 6 receptor antibody prepared through antibody preparation technology. A CHO cell line capable of stably expressing BAT1806 was constructed, and the supernatant was collected and purified through PROTEIN A.

### Example 1 Formulation Preparation

The liquid formulations of the present invention comprising a monoclonal antibody provide a combination of compositions capable of stably preserving the active ingredient, and the formulation formulas of the preferred solutions are shown in Table 1A, Table 1B, Table 1C, Table 1D, Table 1E, and Table 1F.

The method to prepare the antibody formulation provided by the invention comprises the steps of:
(1) dissolving a weighed buffer, a stabilizer and a surfactant in water for injection;
(2) adjusting the liquid prepared in the step (1) with an aqueous sodium hydroxide until the pH is 5-7; preferably, the concentration of aqueous sodium hydroxide being 1 M;
(3) filtering the liquid prepared in the step (2) into an aseptic container; preferably, the pore size of the filter membrane being 0.22 um; and
(4) adding the liquid prepared in the step (3) into an antibody solution.

As an exemplary example, the method of the present invention to prepare 10 L buffer for Formulation A (without antibody BAT1806) was as follows:
The following amounts of ingredients were weighed out: 10.08 g histidine, 7.34 g histidine hydrochloride, 105.33 g arginine hydrochloride, 200 g sucrose, 5 mL polysorbate-80. Sodium hydroxide was dissolved in water for injection to obtain a solution of concentration being 1 M.

The above weighed ingredients were dissolved in about 9 L water for injection, the sequence of adding the ingredients of the formulation does not affect the quality of the formulation, and can be flexibly selected.

After addition of the above formulation ingredients, the pH was adjusted by addition of 1 M sodium hydroxide and finally water for injection was added to reach a constant volume of 10 L, and then the formulation was filtered through a hydrophilic polyvinylidene fluoride filter membrane of 0.22 um pore size, after this, the above 10 L formulation was filtered into an aseptic container.

The method to prepare 10 L Formulation A (containing antibody BAT1806) is as follows:
With the above aseptically filtered buffer for Formulation A (without antibody BAT1806) prepared, the above buffer for Formulation A was added to an antibody concentrate. The antibody concentrate was thawed in a water bath (room temperature) prior to preparing the liquid formulation containing the antibody. Aseptically, the buffer for Formulation A was added to the antibody concentrate containing a total of 200 g antibody along with stirring to obtain the liquid Formulation A containing the antibody of the present invention.

With the liquid formulation containing the antibody prepared, the formulation was packaged for use in vials or pre-filled syringes.

The skilled artisan will also appreciate that the weight, weight to volume ratio, volume to volume ratio referred to herein may be converted to moles and/or molar concentrations using well-known molecular weights of the ingredients. Weight cited herein corresponds to the volume. The skilled artisan will appreciate that the weights may be proportionally adjusted when different formulation volumes are required. For example, 16 L, 14 L, 12 L, 10 L, 5 L of the formulation comprises 1.6, 1.4, 1.2, 1.0, 0.5 times of the cited weights, respectively.

The methods to prepare the other five formulations (Formulation B, Formulation C, Formulation D, Formulation E and Formulation F) are similar to the method to prepare Formulation A, and the weighed reagents and their weights are adjusted accordingly.

### Example 2 Buffer Selection

Studies on antibody stability were performed using a variety of buffer systems.

On the basis of the nature of the antibodies, the inventors had empirically determined a number of buffer systems:
PB, L-His, CB(NMS), PB + HAC, CB + HAC, L-His + NaAC

The meaning of each of the above abbreviations is as follows:
PB: 15 mM phosphate buffer, pH 6.0-6.5
L-His: 10 mM histidine salt buffer, pH 6.0-6.4
CB: 10 mM citrate buffer, pH 6.0-6.4
PB + HAC: 5 mM phosphate + 5 mM sodium acetate mixed buffer, pH 6.0-6.4
CB + HAC: 5 mM citrate + 5 mM sodium acetate mixed buffer, pH 6.0-6.4
L-His + NaAC: 5 mM histidine salt + 5 mM sodium acetate mixed buffer, pH 6.0-6.4

The antibody-buffer system used for the stability analysis contained 20 mg/mL antibody (BAT1806), 10-15 mM buffer (see Table 2), pH 6.0-6.4.

The tendency of the various formulas to change in monomer purity (SEC-HPLC, abbreviated SEC) and charge isomer (IEC-HPLC, abbreviated IEC) at a high temperature 40 °C for 21 days was investigated, see Table 2.

### SEC-HPLC analysis method:

The test article was diluted with water to a 5 mg/mL test article solution. The chromatographic column was TSK-GEL G3000SWXL 7.8 × 300 mM, 5 µm (TOSOH). The mobile phase was 200 mM K3PO4 with 250 mM KCl (pH 7.0). The UV detection wavelength was set at 280nm, the column temperature was 30 °C, the loading quantity of sample was 40 µL (200 µg protein), and the flow was kept for 35 minutes at a rate of 0.5 mL/min. The chromatography was recorded and after integration, the percentage of monomer and aggregate in the sample solution was calculated by area normalization method.

### IEC-HPLC analysis method:

The test article was diluted with water to a 5 mg/mL test article solution. Chromatographic conditions: column, TSK-GEL CM-STAT® 4.6×100 mM, 7 µm (TOSOH); mobile phase, mobile phase A (20 mM ACES, pH 8.0) and mobile phase B (20 mM ACES + 200 mM NaCl, pH 8.0); the loading quantity of sample was 50 µg; the UV detection wavelength was 280 nm; gradient elution was performed according to the elution gradient given below for 45 minutes. The chromatography was recorded. And after integration, the percentage of the main peak, the acidic region and the basic region was respectively calculated using a peak area normalization method. (The chromatography was manually integrated, a base line was drawn at a place where the base line was relatively flat, the integration starting time and the integration ending time were about 8 minutes before and after the retention time of the main peak, vertical lines were drawn at two peak valleys around the main peak, the acidic region was before the main peak that was followed by the basic region (the main peak was followed by basic peak 1, basic peak 2 and basic peak 3 in sequence), and a vertical line was drawn at each peak valley of the basic region.)

**Elution Gradients**

| Time (min) | Mobile Phase A | Mobile Phase B | Flow Rate (mL/min) |
|---|---|---|---|
| 0 | 90% | 10% | 0.6 |
| 5 | 90% | 10% | 0.6 |
| 35 | 70% | 30% | 0.6 |
| 36 | 0% | 100% | 1.0 |
| 39 | 0% | 100% | 1.0 |
| 40 | 90% | 10% | 0.6 |
| 44 | 90% | 10% | 0.6 |

According to the above test results, the best formula was selected as follows: histidine buffer solution, histidine salt + sodium acetate buffer solution; since histidine acts primarily in the pH range of 6.0-6.4, and the pH range for buffering capacity of sodium acetate is around 4.5-5.8, the combination had good results, attributed primarily to the presence of histidine salt, and there were no major differences between the results of histidine salt solely and histidine salt + sodium acetate. If the results of the single buffer and the combined buffer were not significantly different, we would choose the simpler formula. Thus, the most appropriate buffer solution was chosen to be a histidine salt buffer. After high temperature experiments, either SEC aggregation or IEC acidic peaks were significantly lower than those of other buffers or combination buffers.

To select histidine salt concentration, histidine salt buffer was prepared at concentrations ranging from 5-20 mM, with protein concentration of 20 mg/mL, pH 6.0-6.4. The trends of SEC and IEC after 21 days of exposure to 40 °C high temperature were investigated, see Table 3. It can be seen that histidine buffer could play a good buffer protection role in the range of 5-20 mM.

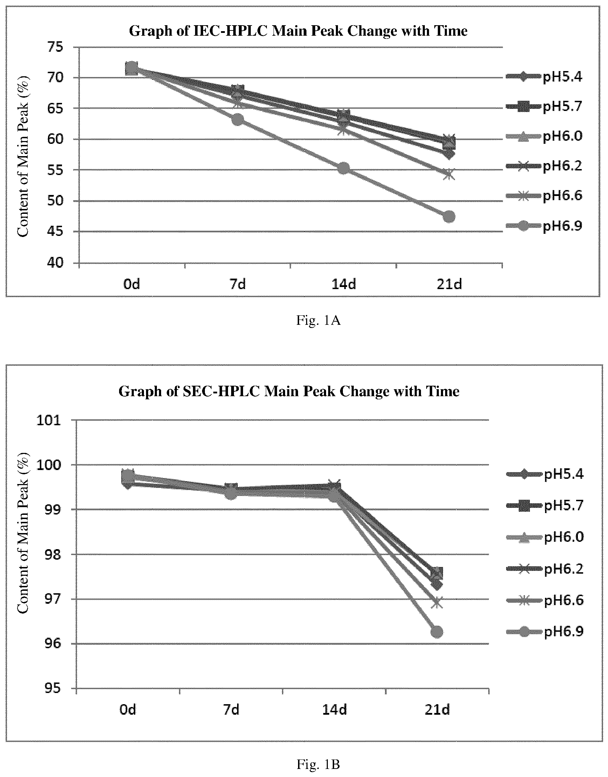

To select the optimal pH of the formulation buffer, histidine salt buffer was prepared at a concentration of 10 mM with a protein concentration of 20 mg/mL in the pH range 5.4-6.9. The trends of SEC and IEC after 21 days of exposure to 40 °C high temperature were investigated, see Figure 1. As can be seen from the mapping of the main peak data of SEC-HPLC and IEC tests, the pH in the range of 5.7-6.2 could play a good buffer protection role as time went by. pH 6.2 is better than pH 6.0 and better than other pH. The results showed that the sample had a higher stability at pH 6.2.

**Table 2. Formulation Buffer Selection at High Temperature (40 °C)**

| Buffer type | PB | L-His | CB(NMS) | PB + HAC | CB + HAC | L-His + NaAC |
|---|---|---|---|---|---|---|
| Buffer concentration (mM) | 15 | 10 | 10 | 10 | 10 | 10 |
| pH | 6.0∼6.4 | 6.0∼6.4 | 6.0∼6.4 | 6.0∼6.4 | 6.0∼6.4 | 6.0∼6.4 |
| Antibody concentration (mg/mL) | 20 | 20 | 20 | 20 | 20 | 20 |
| SEC main peak | 98.38 | 98.59 | 98.36 | 98.51 | 98.38 | 98.47 |
| Day 0 (%) | | | | | | |
| SEC main peak | 95.81 | 97.39 | 97.34 | 97.01 | 96.92 | 97.20 |
| Day 21 (%) | | | | | | |
| SEC aggregation | 0.36 | 0.26 | 0.47 | 0.37 | 0.46 | 0.35 |
| Day 0 (%) | | | | | | |
| SEC aggregation | 1.42 | 0.20 | 0.63 | 0.44 | 0.73 | 0.32 |
| Day 21 (%) | | | | | | |
| IEC main peak | 76.58 | 76.57 | 76.52 | 76.81 | 76.53 | 76.64 |
| Day 0 (%) | | | | | | |
| IEC main peak | 50.56 | 62.24 | 59.60 | 59.39 | 60.56 | 61.92 |
| Day 21 (%) | | | | | | |
| IEC acidic peak | 17.83 | 17.90 | 17.92 | 17.71 | 18.02 | 17.94 |
| Day 0 (%) | | | | | | |
| IEC acidic peak | 43.71 | 30.81 | 33.50 | 33.93 | 32.82 | 31.05 |
| Day 21 (%) | | | | | | |

**Table 3. Histidine Salt Buffer Concentration Selection at High Temperature (40 °C)**

| Histidine salt concentration (mM) | 5 | 10 | 15 | 20 |
|---|---|---|---|---|
| pH | 6.0∼6.4 | 6.0∼6.4 | 6.0∼6.4 | 6.0∼6.4 |
| Antibody concentration (mg/mL) | 20 | 20 | 20 | 20 |
| SEC main peak % | 99.66 | 99.71 | 99.66 | 99.63 |
| Day 0 (%) | | | | |
| SEC main peak % | 96.57 | 97.92 | 97.59 | 97.55 |
| Day 21 (%) | | | | |
| IEC main peak % | 76.43 | 76.52 | 76.49 | 76.51 |
| Day 0 (%) | | | | |
| IEC main peak % | 62.23 | 61.52 | 62.49 | 61.51 |
| Day 21 (%) | | | | |

### Example 3 Stabilizer Selection

In the system for stabilizer selection, antibodies, buffers and stabilizers included are shown in Table 4.

The trends of changes in monomer purity (SEC-HPLC, abbreviated as SEC) and charge isomers (IEC-HPLC, abbreviated as IEC) of the various formulas were investigated under the conditions of 25 °C, 40 °C, 50 °C and light for 3 months, as shown in Table 4. The analysis is described in Example 2.

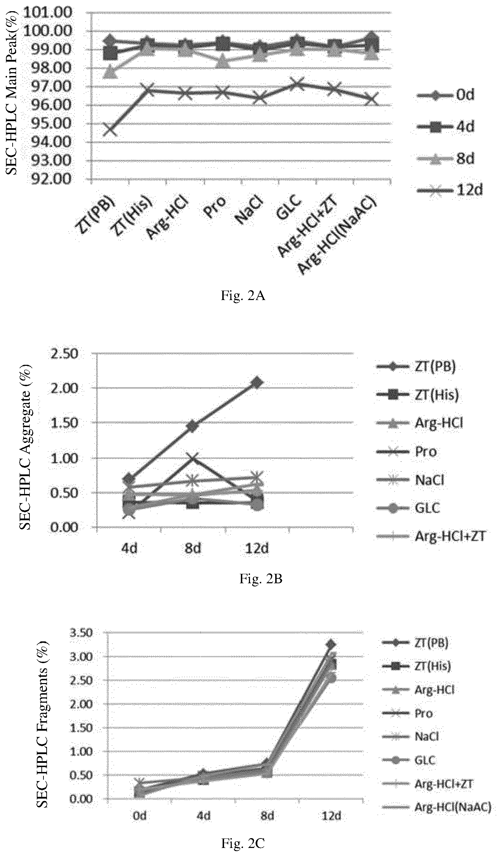

Common stabilizers in antibody formulations are sugar alcohols, amino acids, salts and the like. The stabilizer can stabilize the structure of the antibody and reduce aggregation, degradation of protein molecules and formation of acidic charge isomers under the action of external force. The antibody is relatively stable in composition formulations of different buffer systems such as PB, His, and NaAC and different stabilizers such as sucrose (ZT), mannitol (GLC), arginine hydrochloride (Arg-Hcl), proline (Pro), and sodium chloride (NaCl). The results under the conditions of 40 degree high temperature, 50 degree high temperature and illumination tests are shown in Figures 2-4, respectively. Under the condition of high temperature, the rank of stabilizer performance was as follows: GLC = Arg-HCl + ZT, Arg-HCL, ZT, NaCl, Pro; where SEC main peak was analyzed under the condition of light, the rank of stabilizer performance was as follows: Arg-HCl + ZT ≥ Pro ≥ Arg-HCL ≥ ZT ≥ GLC ≥ NaCl; by analyzing IEC main peak under the condition of light, the rank of performance of stabilizer was as follows: Arg-HCL ≥ Arg-HCl + ZT ≥ Pro ≥ ZT ≥ GLC ≥ NaCl. After an accelerated stability test under 25 °C for 3 months, in histidine salt (His) buffer, the stability of various stabilizers on protein aggregation and degradation turned out to be similar, but in the aspect of reducing the formation of acidic isomers, the rank of stabilizer performance was as follows: Arg-HCL, NaCl, GLC, ZT and Pro in sequence from high to low. By testing the effect of temperature and light on antibody stability, the optimal combination of buffer and stabilizer was selected as follows: 10 mM histidine buffer, 50 mM arginine hydrochloride, 2% sucrose; another combination was as follows: 10 mM histidine salt buffer, 3% mannitol; another combination was as follows: 10 mM histidine salt buffer, 4.2% mannitol; another combination may also be as follows: 10 mM histidine salt buffer, 100 mM sodium chloride.

Furthermore, after an accelerated stability test under 25 °C for 3 months, comparing the histidine salt (His) group without stabilizer with the ZT (His) group with stabilizer, SEC main peaks were respectively 97.06% and 97.76%, and IEC main peaks were respectively 61.94% and 63.17%, when three months expired; it can be seen that in the (histidine salt) His group, with situations with and without stabilizers compared, SEC main peak increased by about 0.7%, IEC main peak increased by about 2%; comparing the PB group without stabilizer and the ZT (PB) group with stabilizer, the SEC main peaks were 95.51% and 96.11%, respectively, and the IEC main peaks were 50.26% and 55.10%, respectively, when three months expired. Thus in the PB group, with situations with and without stabilizers compared, the main SEC peaks increased by about 0.6% and the main IEC peaks increased by about 5%, respectively; without stabilizer and using different buffer systems, the SEC main peaks of the PB group and the (histidine salt) His group were 95.51% and 97.06%, respectively, and the IEC main peaks were 50.26% and 61.94%, respectively, when three months expired. Compared with PB buffer, SEC main peaks of Histidine salt (His) buffer increased by about 1.5% and the main IEC peak increased by about 11%. As can be seen, the selection of an appropriate buffer was very important for maintaining the stability of the antibody, and the buffer played an important role in reducing the structural change of the antibody and increasing the purity of the main peak. See Table 4.

**Table 4. Stabilizer Selection for Humanized Anti-interleukin 6 Receptor Antibody BAT1806 (25 °C, 3 months)**

| Groups | PB | His | ZT (PB) | ZT (His) | Arg-HCl | Pro | NaCl | GLC | GLC | Arg-HCl + ZT | Arg-HCl (NaAc) |
|---|---|---|---|---|---|---|---|---|---|---|---|
| Buffer | 15 mM PB | 10 mM His | 15 mM PB | 10 mM His | 10 mM His | 10 mM His | 10 mM His | 10 mM His | 10 mM His | 10 mM His | 10 mM NaAC |
| Stabilizer | 0 | 0 | 5% ZT | 5% ZT | 100 mM Arg-HCl | 100 mM Pro | 100 mM NaCL | 3% GLC | 4.2% GLC | 50 mM Arg-HCl + 2%ZT | 100 mM Arg-HCl |
| pH | 6.0∼6.4 | 6.0∼6.4 | 6.0∼6.4 | 6.0∼6.4 | 6.0∼6.4 | 6.0∼6.4 | 6.0∼6.4 | 6.0∼6.4 | 6.0∼6.4 | 6.0∼6.4 | 6.0∼6.4 |
| Antibody concentration (mg/mL) | 20 | 20 | 20 | 20 | 20 | 20 | 20 | 20 | 20 | 20 | 20 |
| SEC-HPLC main peak % (0th month) | 99.23 | 99.12 | 99.45 | 99.34 | 99.21 | 99.39 | 99.12 | 99.44 | 99.68 | 99.15 | 99.63 |
| SEC-HPLC main peak % (3rd month) | 95.51 | 97.06 | 96.11 | 97.76 | 97.7 | 97.58 | 97.48 | 97.63 | 97.83 | 97.61 | 97.53 |
| IEC main peak % (0th month) | 73.28 | 73.27 | 74.76 | 72.77 | 72.98 | 73.3 | 73.56 | 72.16 | 73.54 | 73.23 | 70.03 |
| IEC main peak % (3rd month) | 50.26 | 61.94 | 55.10 | 63.17 | 64.93 | 62.98 | 64.74 | 63.62 | 63.98 | 64.06 | 66.01 |

### Example 4 Surfactant Selection

In the surfactant selection system, the antibody, buffer, stabilizer, surfactant included are shown in Table 5.

The number of insoluble particles in each formula after three freeze-thaw cycles under -20 °C to 4 °C was investigated.

The method for determining the amount of insoluble particles was implemented according to General Rule 0903 of the Fourth Volume of the Pharmacopoeia of the People's Republic of China 2015: Insoluble Microparticle Test. After the instrument was cleaned to meet the standard, 4 bottles of test articles were taken in an ultra-clean bench, the outer wall of the container was cleaned by water, the container was carefully flipped for 20 times, the mixture was uniformly mixed, the mixture was allowed to stand for 2 minutes for degassing, the sample was placed on an analyzer, each bottle was measured by the instrument once, and the sample injection amount of each bottle was 3.0 mL. After 4 bottles of samples were sequentially measured, the data of the last 3 bottles were averaged to calculate the total number of particles contained in each bottle of samples.

In antibody formulations, insoluble particles are readily produced upon a freeze/thaw cycle and may be prevented by the addition of a certain amount of surfactant. Common surfactant is nonionic surfactant, such as: polysorbate-20, polysorbate-80 and poloxamer 188. In this example, 0.1% polysorbate-20, 0.05% polysorbate-80, or 0.1% poloxamer 188 was added to the formulation containing buffer and stabilizer, respectively, and the number of insoluble particles after three freeze-thaws under -20 °C to 4 °C was observed. The results showed that all three surfactants can inhibit insoluble particles from being produced by freeze-thaw, and the effects were similar, as shown in Table 5.

**Table 5. Surfactant Selection for Humanized Anti-interleukin 6 Receptor Antibody (-20 °C to 4 °C, 3 freeze-thaw cycles)**

| Groups | Polysorbate-20 | Polysorbate-80 | Poloxamer 188 |
|---|---|---|---|
| Buffer | 10 mM Histidine salt Buffer | 10 mM Histidine salt Buffer | 10 mM Histidine salt Buffer |
| Stabilizer | 4.2% Mannitol | 4.2% Mannitol | 4.2% Mannitol |
| Surfactant | 0.1% Polysorbate-20 | 0.05% Polysorbate-80 | 0.1% poloxamer 188 |
| pH | 6.0∼6.4 | 6.0∼6.4 | 6.0∼6.4 |
| Antibody concentration (mg/mL) | 20 | 20 | 20 |
| Insoluble particles ≥ 10 um (particle/mL) | 8 | 9 | 8 |
| Insoluble particles ≥ 25 um (particle/mL) | 0 | 1 | 1 |

### Example 5 Freeze-thaw Studies

BAT1806 antibody Formulation A, Formulation B, Formulation C, Formulation D with an antibody concentration of 20 mg/mL and BAT1806 antibody Formulation F with a concentration of 50 mg/mL were prepared as described in Example 1. For Formulations A, B, C, D, five cycles of freeze-thaws were repeated under -20 °C to 4 °C, for Formulation F, five cycles of freeze-thaws were repeated under -20 °C to room temperature, the antibody concentration was tested via ELISA method, and the stability of the antibody content in the formulation solution after five cycles of freeze-thaws was investigated. In addition, Formulations A, B, C, D were subjected to fast and slow freeze-thaws under -20 °C to 4 °C, -20 °C to 37 °C, -80 °C to 4 °C, -80 °C to 37 °C for five cycles respectively, and for Formulation F, five freeze-thaw cycles were repeated under -20 °C to room temperature, and changes in the transparency, color, pH, insoluble particles, SEC, IEC, and cell activity were observed.

The ELISA test method is described as follows: antigen recombinant human interleukin 6 receptor (rhIL-6R) (1ug/mL, 100ul per well) was coated; and it was blocked with PBS containing 5% BSA; the BAT1806 antibody Formulations A, B, C and D were respectively diluted by 1×10⁵ times, 100ul per well, and 5 wells were respectively loaded with antibodies diluted to each degree; the initial concentration of the standard was 1ug/mL, and the standard was subjected to 8 gradients of dilution with PBS containing 2% BSA twice; a standard curve was drawn, and a mouse anti-human IgG kappa-HRP was used as a secondary antibody for Elisa test.

The cell activity test method is briefly described as follows: the humanized anti-interleukin 6 receptor antibody BAT1806 antibody standard was used as a standard, the initial concentration was 20ug/mL which was diluted in a gradient manner and added to a 96-well plate, at 50ul per well. The sample to be tested was also diluted according to the standard curve sample dilution method and added to a 96-well plate at 50ul per well. hIL-6 was diluted to 4ng/mL and added to a 96-well plate at 50ul per well. TF-1 cells in logarithmic growth phase were inoculated in a 96-well plate at 100,000 cells/well/100ul, and were incubated for 72 hours in a 37 degree 5% CO₂ incubator. Celltiter Glo reagent was added at 50ul per well. Upon standing for 2 hours in the absence of light at room temperature, microplate data were read via CelltiterGlo method in a microplate reader (Molecular Devices SpectraMax). Four parameters were taken to fit the curve C value (IC50), and according to the equation: the relative specific activity = [IC50 of standard / IC50 of test sample] × 100%, if the calculated results were 80%-125%, the activity of the test sample was considered normal.

The results showed that the recovery rate ranged from 97.5% to 102.4% after five freeze-thaw cycles of the BAT1806 antibody formulations. This indicates that the antibody content was basically unchanged after five freeze-thaw cycles under a frozen condition, see Tables 6A, 6B, 6C, 6D, and 6E. Although the antibody formulation thawed rapidly or slowly from -20 °C or -80 °C and was subjected to freeze-thaw five cycles, the transparency, color, pH, insoluble particles, SEC, IEC and cell activity were not obviously changed, which means that the characteristic of the sample was stable in the repeated freeze-thaw test, no precipitate was generated, the protein was basically not adsorbed, and the characteristics of the samples were not affected. In addition, the SEC-HPLC purity, IEC-HPLC main peak content and other test items of the samples also showed no obvious changes, and activity of the five freeze-thaw cycles were all within an acceptable range.

See Tables 7A, 7B, 7C, 7D, and 7E.

**Table 6A. Antibody Content after Five Freeze-thaw Cycles of Formulation A (-20 °C to 4 °C)**

| Cycles | Antibody Content after One Freeze-thaw Cycle (mg/mL) | Antibody Content after Two Freeze-thaws Cycles (mg/mL) | Antibody Content after Three Freeze-thaw Cycles (mg/mL) | Antibody Content after Four Freeze-thaw Cycles (mg/mL) | Antibody Content after Five Freeze-thaw Cycles (mg/mL) |
|---|---|---|---|---|---|
| 1 | 20.12 | 20.28 | 21.23 | 20.28 | 18.72 |
| 2 | 20.96 | 19.23 | 19.12 | 21.09 | 19.33 |
| 3 | 18.97 | 18.67 | 20.56 | 18.72 | 20.19 |
| 4 | 19.05 | 21.03 | 18.98 | 19.03 | 19.78 |
| 5 | 20.07 | 20.12 | 20.02 | 20.06 | 20.56 |
| Mean | 19.83 | 19.87 | 19.98 | 19.84 | 19.72 |
| SD | 0.83 | 0.93 | 0.95 | 0.96 | 0.72 |
| RE% | 99.2 | 99.3 | 99.9 | 99.2 | 98.6 |

**Table 6B. Antibody Content after Five Freeze-thaw Cycles of Formulation B (-20 °C to 4 °C)**

| Cycles | Antibody Content after One Freeze-thaw Cycle (mg/mL) | Antibody Content after Two Freeze-thaw Cycles (mg/mL) | Antibody Content after Three Freeze-thaw Cycles (mg/mL) | Antibody Content after Four Freeze-thaw Cycles (mg/mL) | Antibody Content after Five Freeze-thaw Cycles (mg/mL) |
|---|---|---|---|---|---|
| 1 | 21.28 | 18.78 | 19.78 | 20.39 | 19.71 |
| 2 | 20.37 | 19.98 | 20.54 | 18.12 | 19.83 |
| 3 | 19.23 | 20.75 | 21.75 | 21.32 | 20.79 |
| 4 | 20.03 | 21.56 | 18.23 | 19.53 | 19.98 |
| 5 | 18.97 | 20.34 | 19.67 | 20.36 | 20.56 |
| Mean | 19.98 | 20.28 | 19.99 | 19.94 | 20.17 |
| SD | 0.93 | 1.03 | 1.29 | 1.20 | 0.47 |
| RE% | 99.9 | 101.4 | 100.0 | 99.7 | 100.9 |

**Table 6C. Antibody Content after Five Freeze-thaw Cycles of Formulation C (-20 °C to 4 °C)**

| Cycles | Antibody Content after One Freeze-thaw Cycle (mg/mL) | Antibody Content after Two Freeze-thaw Cycles (mg/mL) | Antibody Content after Three Freeze-thaw Cycles (mg/mL) | Antibody Content after Four Freeze-thaw Cycles (mg/mL) | Antibody Content after Five Freeze-thaw Cycles (mg/mL) |
|---|---|---|---|---|---|
| 1 | 20.56 | 19.72 | 18.72 | 20.12 | 18.34 |
| 2 | 20.82 | 20.34 | 19.76 | 19.34 | 20.11 |
| 3 | 19.87 | 20.28 | 19.56 | 19.89 | 19.23 |
| 4 | 21.05 | 18.59 | 20.34 | 20.78 | 19.49 |
| 5 | 19.77 | 19.98 | 19.86 | 19.56 | 20.36 |
| Mean | 20.41 | 19.78 | 19.65 | 19.94 | 19.51 |
| SD | 0.57 | 0.71 | 0.59 | 0.56 | 0.80 |
| RE% | 102.1 | 98.9 | 98.2 | 99.7 | 97.5 |

**Table 6D. Antibody Content after Five Freeze-thaw Cycles of Formulation D (-20 °C to 4 °C)**

| Cycles | Antibody Content after One Freeze-thaw Cycle (mg/mL) | Antibody Content after Two Freeze-thaw Cycles (mg/mL) | Antibody Content after Three Freeze-thaw Cycles (mg/mL) | Antibody Content after Four Freeze-thaw Cycles (mg/mL) | Antibody Content after Five Freeze-thaw Cycles (mg/mL) |
|---|---|---|---|---|---|
| 1 | 20.38 | 19.91 | 18.29 | 20.29 | 18.43 |
| 2 | 20.52 | 20.28 | 19.67 | 19.47 | 20.19 |
| 3 | 19.67 | 20.32 | 19.68 | 19.98 | 19.47 |
| 4 | 21.12 | 18.95 | 20.41 | 20.21 | 19.94 |
| 5 | 19.97 | 19.88 | 19.66 | 19.72 | 20.63 |
| Mean | 20.33 | 19.87 | 19.54 | 19.93 | 19.73 |
| SD | 0.55 | 0.55 | 0.77 | 0.34 | 0.84 |
| RE% | 101.7 | 99.3 | 97.7 | 99.7 | 98.7 |

**Table 6E. Antibody Content after Five Freeze-thaw Cycles of Formulation F (-20 °C to room temperature)**

| Cycles | Antibody Content after One Freeze-thaw Cycle (mg/mL) | Antibody Content after Two Freeze-thaw Cycles (mg/mL) | Antibody Content after Three Freeze-thaw Cycles (mg/mL) | Antibody Content after Four Freeze-thaw Cycles (mg/mL) | Antibody Content after Five Freeze-thaw Cycles (mg/mL) |
|---|---|---|---|---|---|
| 1 | 51.10 | 51.40 | 51.70 | 50.80 | 51.20 |
| 2 | 51.32 | 51.80 | 50.50 | 50.66 | 51.10 |
| 3 | 51.12 | 50.23 | 51.21 | 51.22 | 50.68 |
| 4 | 50.81 | 51.52 | 51.01 | 51.10 | 50.80 |
| 5 | 51.79 | 50.31 | 50.83 | 50.68 | 51.02 |
| Mean | 51.22 | 51.05 | 51.05 | 50.89 | 50.96 |
| SD | 0.32 | 0.65 | 0.40 | 0.23 | 0.19 |
| RE% | 102.4 | 102.1 | 102.1 | 101.7 | 101.9 |

**Table 7A. Results of Five Rapid/Slow Freeze-thaw Tests for Formulation A**

| Test parameters | No freeze-thaw | -20 °C to 4 °C Five cycles | -20 °C to 37 °C Five cycles | -80 °C to 4 °C Five cycles | -80 °C to 37 °C Five cycles |
|---|---|---|---|---|---|
| Transparency | 25.0 | 24.8 | 25.2 | 25.1 | 25.3 |
| Color | ≤Y5 | ≤Y5 | ≤Y5 | ≤Y5 | ≤Y5 |
| pH | 6.0∼6.4 | 6.0∼6.4 | 6.0∼6.4 | 6.0∼6.4 | 6.0∼6.4 |
| Insoluble particles ≥10 um (particle/mL) | 9 | 11 | 13 | 10 | 11 |
| Insoluble particles ≥25 um (particle/mL) | 1 | 2 | 1 | 3 | 1 |
| SEC-main peak % | 99.15 | 98.64 | 98.55 | 98.77 | 98.42 |
| IEC- main peak % | 73.23 | 73.21 | 73.26 | 73.17 | 73.29 |
| Cell activity (%) | 98.2% | 107.9% | 104.3% | 97.2% | 95.8% |

**Table 7B. Results of Five Rapid/Slow Freeze-thaw Tests for Formulation B**

| Test parameters | No freeze-thaw | - 20 °C-4 °C Five cycles | -20 °C to 37 °C Five cycles | -80 °C to 4 °C Five cycles | -80 °C to 37 °C Five cycles |
|---|---|---|---|---|---|
| Transparency | 25.0 | 24.8 | 25.2 | 25.1 | 25.3 |
| Color | ≤Y5 | ≤Y5 | ≤Y5 | ≤Y5 | ≤Y5 |
| pH | 6.0∼6.4 | 6.0∼6.4 | 6.0∼6.4 | 6.0∼6.4 | 6.0∼6.4 |
| Insoluble particles ≥10 um (particle/mL) | 8 | 10 | 12 | 9 | 10 |
| Insoluble particles ≥25 um (particle/mL) | 2 | 1 | 1 | 2 | 3 |
| SEC-main peak % | 99.44 | 98.87 | 98.79 | 98.82 | 98.58 |
| IEC- main peak % | 72.16 | 72.09 | 72.19 | 72.17 | 72.10 |
| Cell activity (%) | 110.2% | 104.9% | 94.6% | 102.5% | 98.9% |

**Table 7C. Results of Five Rapid/Slow Freeze-thaw Tests for Formulation C**

| Test parameters | No freeze-thaw | -20 °C to 4 °C Five cycles | -20 °C to 37 °C Five cycles | -80 °C to 4 °C Five cycles | -80 °C to 37 °C Five cycles |
|---|---|---|---|---|---|
| Transparency | 25.0 | 24.8 | 25.2 | 25.1 | 25.3 |
| Color | ≤Y5 | ≤Y5 | ≤Y5 | ≤Y5 | ≤Y5 |
| pH | 6.0∼6.4 | 6.0∼6.4 | 6.0∼6.4 | 6.0∼6.4 | 6.0∼6.4 |
| Insoluble particles≥10 um (particle/mL) | 10 | 12 | 10 | 9 | 11 |
| Insoluble particles≥25 um (particle/mL) | 1 | 2 | 1 | 1 | 1 |
| SEC-main peak % | 99.12 | 98.60 | 98.57 | 98.52 | 98.62 |
| IEC-main peak % | 73.56 | 73.62 | 73.43 | 73.82 | 73.49 |
| Cell activity (%) | 105.8% | 99.7% | 112.5% | 108.3% | 95.7% |

**Table 7D. Results of Five Rapid/Slow Freeze-thaw Tests for Formulation D**

| Test parameters | No freeze-thaw | - 20 °C-4 °C Five cycles | -20 °C to 37 °C Five cycles | -80 °C to 4 °C Five cycles | -80 °C to 37 °C Five cycles |
|---|---|---|---|---|---|
| Transparency | 24.8 | 25.0 | 24.9 | 25.2 | 25.3 |
| Color | ≤Y5 | ≤Y5 | ≤Y5 | ≤Y5 | ≤Y5 |
| pH | 6.0∼6.4 | 6.0∼6.4 | 6.0∼6.4 | 6.0∼6.4 | 6.0∼6.4 |
| Insoluble particles≥10 um (particle/mL) | 9 | 13 | 8 | 9 | 10 |
| Insoluble particles≥25 um (particle/mL) | 1 | 2 | 2 | 1 | 2 |
| SEC-main peak % | 98.21 | 97.60 | 98.35 | 98.92 | 97.62 |
| IEC- main peak % | 73.78 | 73.25 | 73.33 | 73.62 | 73.94 |
| Cell activity (%) | 103.1% | 95.7% | 102.8% | 98.3% | 92.8% |

**Table 7E. Results of Five -20 °C to Room Temperature Freeze-thaw Tests for Formulation F**

| Test condition | | -20 °C frozen for 24h, thawed at room temperature, allowed to stand for 24h (one freeze-thaw cycle) | | | | | |
|---|---|---|---|---|---|---|---|
| Test item | | 0^{th} cycle | 1^{st} cycle | 2^{nd} cycle | 3^{rd} cycle | 4^{th} cycle | 5^{th} cycle |
| Solution characteristics | | clear no foreign matter | clear no foreign matter | clear no foreign matter | clear no foreign matter | clear no foreign matter | clear no foreign matter |
| pH | | 6.26 | 6.33 | 6.33 | 6.32 | 6.32 | 6.32 |
| SEC-HPL C (%) | aggregate | 0.24 | 0.26 | 0.25 | 0.25 | 0.25 | 0.25 |
| | purity of monomer | 99.66 | 99.63 | 99.65 | 99.65 | 99.64 | 99.64 |
| | fragment | 0.10 | 0.11 | 0.10 | 0.10 | 0.10 | 0.10 |
| IEC-HPL C (%) | acidic region | 25.40 | 25.99 | 25.70 | 25.63 | 25.78 | 25.14 |
| | main peak | 70.34 | 69.77 | 70.28 | 70.59 | 70.38 | 71.19 |
| | basic peaks | 4.26 | 4.24 | 4.03 | 3.78 | 3.84 | 3.67 |
| Cell activity (%) | | 118.2% | 101.9% | 87.5% | 107.7% | 85.7% | 73.7% |
| CE-SDS (non-reduced, %) | | 97.03 | 97.04 | 96.76 | 97.22 | 97.02 | 96.94 |
| CE-SDS (reduced, %) | | 0.48 | 0.45 | 0.52 | 0.48 | 0.47 | 0.46 |
| Insoluble particles (particle/mL) | ≥10 µm | 11.33 | N/A | N/A | N/A | N/A | N/A |
| | ≥25 µm | 0.33 | N/A | N/A | N/A | N/A | N/A |

### Example 6 Microbial Studies

Microbial studies of pharmaceutical formulations (Formulations A, B, C, D) are required to determine whether the formulations support microbial growth. The overall microbial growth of the inoculated formulations was examined by directly inoculating the aseptic formulations with microorganisms (e.g., *Staphylococcus aureus,* ATDD-NO: 6538p, *Candida albicans*, ATDD-NO: 10231, *Aspergillus niger,* ATDD-NO: 16404, environmental isolate) at low levels (NMT100 cfu/mL). The evaluation indicators included mainly the change in turbidity and the number of microorganisms under the microscope, where the lack of turbidity was an indicator of no overall growth, and was tested in the inoculated containers after 14 days. In addition, microorganisms could not be re-isolated from these vessels. Table 8 showed that the formulations did not support microbial growth if stored for 14 days at room temperature 20-25 °C.

**Table 8. Test about Microorganisms in Humanized Anti-interleukin 6 Receptor Antibody BAT1806 Formulation**

| Test parameters | | Number of microorganisms cfu/mL | | | | Turbidity/NTU | | |
|---|---|---|---|---|---|---|---|---|
| Formulation type | Formula -tion A | Formula -tion B | Formula -tion C | Formula -tion D | Formula -tion A | Formula -tion B | Formula -tion C | Formula -tion D |
| *Staphylococcus aureus* | 0 | 0 | 0 | 0 | - | - | - | - |
| *Candida albicans* | 0 | 0 | 0 | 0 | - | - | - | - |
| *Aspergillus niger* | 0 | 0 | 0 | 0 | - | - | - | - |
| *Pseudomonas aeruginosa* | 0 | 0 | 0 | 0 | - | - | - | - |

| | | | | | | | | |
|---|---|---|---|---|---|---|---|---|
| "-" means that turbidity did not change | | | | | | | | |

### Example 7 Stability Studies under the Acceleration Conditions

Pharmaceutical Formulations A, B, C, D, E and F were formulated according to the method of Example 1. The six formulations were placed in a biochemical incubator for 6 months under the condition of (25±2) °C and sampled at the end of the 0th, 1st, 2nd, 3rd and 6th months, respectively. The samples were examined with regard to monomer purity (SEC-HPLC), charge isomer (IEC-HPLC), non-reducing capillary gel electrophoresis (CE-SDS-NR), insoluble microparticles and cell activity according to the major stability examination items (see Table 9). In addition, for Formulation F, the solution characteristic, pH, and antibody content under the acceleration conditions were also investigated.

The monomer purity (SEC-HPLC), charge isomer (IEC-HPLC) were determined as described in Example 2. Analysis method of (CE-SDS-NR): the recombinant sample was diluted to 4 mg/mL with water, 25µl of the diluted sample solution was taken, 70µl CE-SDS sample buffer and 5µl 0.25M iodoacetamide aqueous solution were added, the solution was mixed evenly and heated in 65 °C water bath for 4 minutes, and transferred to a sample bottle to obtain a reference working solution. The sample was introduced with -5.0kV voltage for 10s, then separated and analyzed with -15kV voltage. The chromatography was recorded and integrated automatically within 14-35min. The main peak was the chromatographic peak of the whole antibody protein, the impurity peak before the main peak was the chromatographic peak of Fraction (including L peak, H peak, HL, HH and HHL peaks), and the percentage content of the monomer peak was calculated by an area normalization method. The method of analyzing insoluble microparticles is described in Example 4. The method of analyzing cell activity is described in Example 5.

The results are shown in Table 9. It can be seen from Table 9 that under the acceleration conditions, the monomer purity decreased with time over 6 months, indicating that aggregates were produced under the acceleration conditions, but the SEC main peak of the antibody decreased by no more than 2.28%; with respect to IEC-HPLC, under the condition of 25 °C, the content of IEC main peaks of antibody decreased, and the degradation trend was basically the same, and the IEC main peak decreased by no more than 16.54%; the CE-SDS-NR main peak decreased by no more than 2.1%, insoluble particles were far lower than qualified standards, cell active substances did not change obviously, which indicates that the antibody formulations can still maintain stability within 6 months at room temperature.

The solution characteristics, pH and antibody concentration results for Formulation E are shown in Table 10. From the data in the table, it can be seen that, compared with Day 0, after 6 months under the condition of 25 °C, the sample was still clear and transparent liquid, no visible foreign matter appeared, the pH and antibody concentration of the sample did not change significantly, indicating that the sample was stable in the acceleration test.

**Table 9. Stability Studies under Acceleration Conditions (25±2) °C**

| **SEC-HPLC main peak %** | | | | | |
|---|---|---|---|---|---|
| | 0 month | 1 month | 2 months | 3 months | 6 months |
| Formulation A | 99.45 | 99.38 | 98.92 | 98.57 | 98.21 |
| Formulation B | 99.23 | 99.15 | 98.83 | 98.49 | 98.13 |
| Formulation C | 99.39 | 99.27 | 99.84 | 99.42 | 98.35 |
| Formulation D | 99.31 | 99.21 | 98.93 | 98.52 | 98.21 |
| Formulation E | 99.23 | 98.46 | 98.26 | 98.43 | 96.95 |
| Formulation F | 99.66 | 99.55 | 98.58 | 99.38 | 97.47 |

| **IEC-HPLC main peak %** | | | | | |
|---|---|---|---|---|---|
| | 0 month | 1 month | 2 months | 3 months | 6 months |
| Formulation A | 76.82 | 74.90 | 70.36 | 65.25 | 61.23 |
| Formulation B | 76.75 | 74.23 | 70.18 | 65.78 | 60.98 |
| Formulation C | 76.84 | 74.82 | 69.97 | 65.92 | 61.21 |
| Formulation D | 76.68 | 74.32 | 70.12 | 64.28 | 60.12 |
| Formulation E | 73.46 | 71.70 | 69.55 | 72.17 | 61.51 |
| Formulation F | 70.34 | 68.28 | 66.5 | 65.68 | 62.54 |

| **CE-SDS-NR main peak** % | | | | | |
|---|---|---|---|---|---|
| | 0 month | 1 month | 2 months | 3 months | 6 months |
| Formulation A | 97.2 | 96.9 | 96.3 | 96.1 | 95.7 |
| Formulation B | 97.1 | 96.7 | 96.5 | 96.2 | 95.5 |
| Formulation C | 96.9 | 96.5 | 95.9 | 95.6 | 95.1 |
| Formulation D | 97.2 | 96.3 | 95.9 | 95.2 | 95.1 |
| Formulation E | 95.5 | 95.75 | 95.81 | 95.55 | 94.0 |
| Formulation F | 97.03 | 96.78 | 96.56 | 96.31 | 96.10 |

| **Insoluble particles≥10 um (particle/mL)** | | | | | |
|---|---|---|---|---|---|
| | 0 month | 1 month | 2 months | 3 months | 6 months |
| Formulation A | 13 | 12 | 14 | 13 | 11 |
| Formulation B | 15 | 13 | 17 | 16 | 15 |
| Formulation C | 11 | 14 | 12 | 11 | 14 |
| Formulation D | 12 | 11 | 13 | 12 | 11 |
| Formulation E | 209 | 29 | 19 | 43 | 21 |
| Formulation F | 12 | 10 | 11 | 10 | 30 |

| **Insoluble particles≥25 um (particle/mL)** | | | | | |
|---|---|---|---|---|---|
| | 0 months | 1 month | 2 months | 3 months | 6 months |
| Formulation A | 0 | 1 | 2 | 2 | 1 |
| Formulation B | 1 | 2 | 2 | 1 | 2 |
| Formulation C | 0 | 1 | 1 | 0 | 1 |
| Formulation D | 0 | 0 | 1 | 0 | 1 |
| Formulation E | 3 | 0 | 0 | 0 | 0 |
| Formulation F | 1 | 2 | 1 | 0 | 0 |

| **Cell activity %** | | | | | |
|---|---|---|---|---|---|
| | 0 months | 1 month | 2 months | 3 months | 6 months |
| Formulation A | 98.2 | 91.7 | 121.2 | 105.3 | 108.4 |
| Formulation B | 110.2 | 99.0 | 95.9 | 98.0 | 126.4 |
| Formulation C | 105.8 | 107.1 | 112.9 | 92.8 | 99.7 |
| Formulation D | 97.8 | 95.2 | 105.3 | 95.8 | 97.2 |
| Formulation E | 98.4 | 105.6 | 104.9 | 115.2 | 103.7 |
| Formulation F | 118.2 | 84.9 | 101.3 | 113.0 | 100.9 |

**Table 10. Stability Studies under Acceleration Conditions for Formulation E (25±2) °C**

| Test condition | 25 °C | | | | |
|---|---|---|---|---|---|
| Test item | 0 M | 1 M | 2 M | 3 M | 6 M |
| Solution characteristics | clear no foreign matter | clear no foreign matter | clear no foreign matter | clear no foreign matter | clear no foreign matter |
| pH | 6.24 | 6.16 | 6.15 | 6.12 | 6.12 |
| Antibody concentration (mg/mL) | 22.82 | 22.30 | 22.53 | 21.57 | 22.30 |

From the data in the table above, it can be seen that, for Formulation E, compared with Day 0, after 6 months under the condition of 25 °C, the sample was still clear and transparent liquid, no visible foreign matter appeared, the pH and antibody concentration of the sample did not change significantly, indicating that the sample was stable in the acceleration test.

The SEC, IEC and CE (non-reduced) trend profiles for Formulations E and F at 25 °C under the condition of acceleration are shown in sequence in Figures 5A-5C. Comparing Formulations E and F (both are of the same formula, with two different antibody concentrations), the degradation trends of the main peaks of SEC, IEC and CE (non-reduced) were the same after 6 months under 25 °C in the acceleration test, the monomer purity of the SEC main peak was more than 96.5%, the CE (non-reduced) main peak was more than 94%, and the pH, particles and biological activity were all in accordance with the quality standards, which indicated that the formula was stable with regard to different concentrations of antibody.

### Example 8 Long Term Stability Studies

Pharmaceutical Formulations A, B, C, D were formulated according to the method of Example 1. The above four formulations were placed under 4 °C for 36 months, and sampled at the end of the 0^{th}, 3^{rd}, 6^{th}, 9^{th}, 12^{th}, 24^{th}, 30^{th} and 36^{th} months, respectively. The samples were examined with regard to purity (SEC-HPLC), charge isomers (IEC-HPLC), non-reducing capillary gel electrophoresis (CE-SDS-NR), insoluble microparticles and cellular activity. The results are shown in Table 11. As can be seen from Table 11, the antibody formulations of the present invention maintain long-term stability under 4 °C and were stable at the 36^{th} month.

**Table 11. Long Term Stability Studies 4 °C**

| **SEC-HPLC main peak %** | | | | | | | |
|---|---|---|---|---|---|---|---|
| | 0 month | 3 months | 6 months | 9 months | 12 months | 24 months | 36 months |
| Formulation A | 99.45 | 99.47 | 99.28 | 99.23 | 99.52 | 98.79 | 98.21 |
| Formulation B | 99.23 | 99.34 | 99.29 | 99.41 | 99.19 | 98.72 | 98.11 |
| Formulation C | 99.39 | 99.27 | 99.41 | 99.28 | 99.37 | 99.12 | 98.24 |
| Formulation D | 99.21 | 99.25 | 99.18 | 99.15 | 99.05 | 98.53 | 98.23 |

| **IEC-HPLC main peak %** | | | | | | | |
|---|---|---|---|---|---|---|---|
| | 0 month | 3 months | 6 months | 9 months | 12 months | 24 months | 36 months |
| Formulation A | 76.82 | 75.51 | 74.23 | 73.19 | 72.12 | 70.83 | 69.79 |
| Formulation B | 76.75 | 75.51 | 74.28 | 74.12 | 72.97 | 71.12 | 70.29 |
| Formulation C | 76.84 | 75.98 | 75.12 | 74.23 | 73.11 | 71.98 | 70.12 |
| Formulation D | 76.79 | 75.62 | 74.32 | 73.57 | 72.19 | 70.52 | 68.91 |

| **CE-SDS-NR main peak %** | | | | | | | |
|---|---|---|---|---|---|---|---|
| | 0 month | 3 months | 6 months | 9 months | 12 months | 24 months | 36 months |
| Formulation A | 97.2 | 97.1 | 97.3 | 97.2 | 96.9 | 96.1 | 95.2 |
| Formulation B | 97.1 | 97.2 | 97.5 | 96.7 | 97.1 | 96.3 | 95.1 |
| Formulation C | 96.9 | 97.2 | 97.1 | 96.5 | 96.8 | 95.9 | 95.0 |
| Formulation D | 97.4 | 97.1 | 97.2 | 96.7 | 97.1 | 96.0 | 95.1 |

| **Insoluble particles≥10 um (particle/mL)** | | | | | | | |
|---|---|---|---|---|---|---|---|
| | 0 month | 3 months | 6 months | 9 months | 12 months | 24 months | 36 months |
| Formulation A | 13 | 10 | 12 | 15 | 9 | 12 | 15 |
| Formulation B | 15 | 17 | 11 | 12 | 16 | 11 | 17 |
| Formulation C | 11 | 12 | 9 | 14 | 11 | 12 | 8 |
| Formulation D | 13 | 15 | 11 | 12 | 11 | 13 | 12 |

| **Insoluble particles≥25 um (particle/mL)** | | | | | | | |
|---|---|---|---|---|---|---|---|
| | 0 month | 3 months | 6 months | 9 months | 12 months | 24 months | 36 months |
| Formulation A | 0 | 1 | 0 | 0 | 1 | 0 | 0 |
| Formulation B | 1 | 1 | 0 | 0 | 0 | 0 | 1 |
| Formulation C | 0 | 1 | 0 | 0 | 1 | 0 | 0 |
| Formulation D | 0 | 0 | 1 | 1 | 0 | 1 | 1 |

| **Cell activity %** | | | | | | | |
|---|---|---|---|---|---|---|---|
| | 0 month | 3 months | 6 months | 9 months | 12 months | 24 months | 36 months |
| Formulation A | 98.2 | 88.6 | 101.4 | 80.0 | 89.5 | 99.8 | 81.0 |
| Formulation B | 110.2 | 97.5 | 117.6 | 111.8 | 91.8 | 83.2 | 92.3 |
| Formulation C | 105.8 | 90.3 | 82.0 | 98.6 | 116.0 | 87.7 | 107.4 |
| Formulation D | 97.8 | 102.4 | 93.7 | 98.2 | 112.3 | 95.2 | 98.7 |

### Example 9 Stability Studies under the Conditions of High Temperature and Light

Formulations E and F of Example 1 were subjected to high temperature and light studies to investigate the stability of the formulations under high temperature and light conditions. The test method is described in the other examples.

**Table 12. Test Results under High Temperature Conditions for Formulation E**

| Test condition | | | 40 °C | | | | |
|---|---|---|---|---|---|---|---|
| Test item | | | 0d | 7d | 14d | 21d | 28d |
| Solution characteristics | | | clear no foreign matter | clear no foreign matter | clear no foreign matter | clear no foreign matter | clear no foreign matter |
| pH | | | 6.24 | 6.25 | 6.16 | 6.14 | 6.13 |
| Antibody concentration (mg/mL) | | | 22.82 | 22.77 | 22.55 | 22.19 | 22.11 |
| SEC-HPLC (%) | aggregate | | 0.43 | 0.40 | 0.46 | 0.52 | 0.53 |
| | purity of monomer | | 99.23 | 99.38 | 97.95 | 97.24 | 96.71 |
| | fragment | | 0.34 | 0.22 | 1.59 | 2.24 | 2.76 |
| IEC-HPLC (%) | acidic region | | 22.21 | 26.23 | 31.32 | 36.83 | 40.70 |
| | main peak | | 73.46 | 69.05 | 64.27 | 57.58 | 54.36 |
| | basic peaks | | 4.33 | 4.72 | 4.41 | 5.59 | 4.95 |
| Cell activity (%) | | | 98.4% | 102.7% | 90.9% | 102.7% | 95.7% |
| CE-SDS (non-reduced, %) | | | 95.5 | 95.2 | 93.76 | 93.82 | 92.58 |
| **Insoluble** particles (particle/mL) | | ≥10 µm | 209 | 131 | 137 | 11 | 54 |
| | | ≥25 µm | 3 | 4 | 8 | 0 | 1 |

From the data in the above table, it can be seen that after 28 days under the condition of 40 °C, compared with Day 0, the sample was still clear and transparent liquid, no visible foreign matter appeared, and the pH and antibody concentration of the sample had no obvious change, indicating that the sample was stable in the forced degradation test. In the aspect of SEC-HPLC, monomer purity decreased with time under the condition of 40 °C for 28 days, indicating the formation of aggregate at high temperature; in the aspect of IEC-HPLC, under the condition of 40 °C, the content of IEC main peaks of antibody all decreased, and the degradation trends were basically the same; as can be seen from the insoluble particle data, the test results showed some fluctuation in different days, but the data did not show a gradually increasing trend, and the activities were in a qualified range.

**Table 13. Test Results under the Condition of Light for Formulation E**

| Test condition | | 4000x1 light | | |
|---|---|---|---|---|
| Test item | | 0d | 7d | 14d |
| Solution characteristics | | clear no foreign matter | clear no foreign matter | clear no foreign matter |
| pH | | 6.24 | 6.19 | 6.15 |
| Antibody concentration (mg/mL) | | 22.82 | 22.70 | 22.55 |
| SEC-HPLC (%) | aggregate | 0.43 | 0.72 | 0.74 |
| | purity of monomer | 99.23 | 99.17 | 98.92 |
| | fragment | 0.34 | 0.12 | 0.35 |
| IEC-HPLC (%) | acidic region | 22.21 | 27.67 | 27.41 |
| | main peak | 73.46 | 68.11 | 68.91 |
| | basic peaks | 4.33 | 4.21 | 3.68 |
| Cell activity (%) | | 98.4% | 105.7% | 80.8% |
| CE-SDS (non-reduced, %) | | 95.5 | 94.86 | 94.39 |
| Insoluble particles (particle/mL) | ≥10 µm | 209 | 145 | 137 |
| | ≥25 µm | 3 | 2 | 8 |

From the data in the table above, it can be seen that after 14 days under the condition of 4000x1 light, compared with Day 0, the sample was still clear and transparent liquid, no visible foreign matter appeared, and the pH and antibody concentration of the sample did not change significantly, indicating that the sample was stable under the light condition. In terms of SEC-HPLC, the change in monomer purity was less obvious; in terms of IEC-HPLC, under the condition of 4000x1 light, the IEC main peaks of antibody decreased, and the degradation trends were basically the same; from the data about insoluble particles, it can be seen that the results of different days showed a certain fluctuation, but the data did not show a gradually increasing trend, the activity was in a qualified range, far below the standard.

SEC trends of Formulation E under the conditions of high temperature and light are shown in Figure 6A, IEC trends are shown in Figure 6B, and the CE (non-reduced) trends are shown in Figure 6C. With the results of SEC, IEC and CE (non-reduced) combined, the main peaks declined in a uniform trend after 14 days of high temperature and light, and aggregates were formed in each case under the conditions of high temperature and light, thus the sample were supposed to be preserved in a low temperature and from light.

**Table 14. Test Results under the Condition of High Temperature and Light for Formulation F**

| Test condition | | | 40 °C | | 4000lx | |
|---|---|---|---|---|---|---|
| Test item | | 0d | 7d | 14d | 7d | 14d |
| Solution characteristics | | clear no foreign matter | Clear no foreign matter | clear no foreign matter | clear no foreign matter | clear no foreign matter |
| pH | | 6.26 | 6.26 | 6.25 | 6.22 | 6.21 |
| Antibody concentration (mg/mL) | | 50.12 | 51.05 | 52.22 | 49.45 | 50.76 |
| SEC-HPLC (%) | aggregate | 0.24 | 0.30 | 0.35 | 1.47 | 1.73 |
| | purity of monomer | 99.66 | 99.48 | 99.30 | 98.38 | 98.08 |
| | fragment | 0.10 | 0.22 | 0.15 | 0.34 | 0.19 |
| IEC-HPLC (%) | acidic region | 25.40 | 29.58 | 34.05 | 31.95 | 33.77 |
| | main peak | 70.34 | 65.63 | 61.14 | 63.68 | 61.78 |
| | basic peaks | 4.26 | 4.78 | 4.82 | 4.37 | 4.45 |
| Cell activity (%) | | 118.2% | 102.6% | 84.8% | 90.9% | 116.3% |
| CE-SDS (non-reduced, %) | | 97.03 | 96.94 | 95.64 | 97.16 | 95.83 |
| CE-SDS (reduced, %) | | 0.48 | 0.48 | 0.46 | 0.49 | 0.46 |
| Insoluble particles (particle/mL) | ≥10 µm | 11.33 | 3.33 | 17.67 | 4.67 | 6 |
| | ≥25 µm | 0.33 | 1.33 | 1.33 | 1 | 0 |

From the data in the above table, it can be seen that, after the formulation was placed for 14 days under the condition of 40 °C or 4000 lx light, compared with Day 0, the samples were still clear and transparent liquid, no visible foreign matter appeared, the pH and antibody concentration of the stock solution had no obvious change, which indicated that in the forced degradation test, the sample was relatively stable, no precipitate existed, the protein was basically not adsorbed, and the characteristics of the sample were not affected. In terms of SEC-HPLC, there was no change in monomer purity when the samples were kept at 40 °C for 14 days, and the monomer purity decreased with time under the condition of light, indicating that the antibody was easier to aggregate under the light condition than under high temperature; in terms of IEC-HPLC, under the condition of 40 °C or light, IEC main peaks of antibody decreased, and the degradation trends were basically the same; As can be seen from the insoluble particle data, the test results of different days showed some fluctuation, but the data did not show a gradually increasing trend, and the phenomenon of sudden increase of particles did not appear, far below the qualification standard.

The experiments showed that the antibody formulations with different concentrations all have certain stability under the conditions of high temperature and light.

### Example 10 Stability Studies under the Condition of Oscillation

Experimental conditions: Formulation F was flatwise placed at 200rpm and oscillated at room temperature for 48h. The solution characteristics, antibody content, SEC, IEC, bio-activity, CE-SDS (reduced and non-reduced) and insoluble particles were tested at regular intervals. The test method is described with reference to other examples.

**Table 15. Test Results under Oscillation Condition**

| Test condition | | | 200rpm, flatwise placed, oscillation test under room temperature | | | | | |
|---|---|---|---|---|---|---|---|---|
| Test item | | | 0H | 2H | 4H | 8H | 24H | 48H |
| Solution characteristics | | | clear no foreign matter | clear no foreign matter | clear no foreign matter | clear no foreign matter | clear no foreign matter | clear no foreign matter |
| Antibody concentration (mg/mL) | | | 50.50 | 51.30 | 51.12 | 49.31 | 50.14 | 52.12 |
| (UV A₃₄₀nm) | | | 0.285 | 0.301 | 0.192 | 0.171 | 0.234 | 0.333 |
| SEC-HPLC (%) | aggregate | | 0.24 | 0.25 | 0.24 | 0.27 | 0.25 | 0.24 |
| | purity of monomer | | 99.66 | 99.66 | 99.66 | 99.63 | 99.65 | 99.67 |
| | fragment | | 0.10 | 0.10 | 0.10 | 0.10 | 0.10 | 0.10 |
| IEC-HPLC (%) | acidic region | | 25.40 | 25.96 | 25.32 | 26.05 | 25.76 | 25.20 |
| | main peak | | 70.34 | 70.12 | 70.91 | 70.20 | 70.73 | 71.20 |
| | basic peaks | | 4.26 | 3.92 | 3.77 | 3.76 | 3.51 | 3.60 |
| Bio-activity (%) | | | 118.2% | 104.3% | 125.0% | 107.8% | 86.1% | 101.4% |
| CE-SDS (non-reduced, %) | | | 94.63 | 97.64 | 97.15 | 97.85 | 97.03 | 97.71 |
| CE-SDS (reduced, %) | | | 0.48 | 0.43 | 0.45 | 0.44 | 0.45 | 0.45 |
| Insoluble particles (particle/mL) | | ≥10 µm | 11.33 | N/A | N/A | N/A | N/A | N/A |
| | | ≥25 µm | 0.33 | N/A | N/A | N/A | N/A | N/A |

From the data in the table above, it can be seen that, after 48 hours of oscillation, compared with the data at 0H, the sample was still clear and transparent liquid, no visible foreign matter appeared, and the pH value and antibody concentration of the sample did not change significantly, indicating that the stability of the sample was good in the oscillation test. In addition, the SEC-HPLC purity, IEC-HPLC main peak content and other test items showed no obvious change either, and the activity was within an acceptable range.

### Example 11 Protein Sequence of the Humanized Anti-interleukin 6 Receptor Antibody BAT1806

A humanized anti-interleukin 6 receptor antibody BAT1806 for the treatment of IL-6 related diseases was expressed in CHO cells by genetic engineering means and obtained by purifying through a series of standard chromatographic steps. BAT1806 is an IgG antibody, with a molecular weight of 145 kDa; each heavy chain contains 449 amino acids, with a molecular weight of 53 kDa, and the amino acid sequence of the heavy chain is shown in Table 16; each light chain contains 214 amino acids, with a molecular weight of 24 kDa, and the amino acid sequence of the light chain is shown in Table 17.

**Table 16. Heavy Chain Amino Acid Sequence of Humanized Anti-interleukin 6 Receptor Antibody BAT1806**

| | |
|---|---|
| SEQ ID NO.1 | 5`>QVQLQESGPGLVRPSQTLSLTCTVSGYSITSDHAWSWVRQPPGRGLEW IGYISYSGITTYNPSLKSRVTMLRDTSKNQFSLRLSSVTAADTAVYYCARS LARTTAMDYWGQGSLVTVSSASTKGPSVFPLAPSSKSTSGGTAALGCLVKD YFPEPVTVSWNSGALTSGVHTFPAVLQSSGLYSLSSVVTVPSSSLGTQTYI CNVNHKPSNTKVDKKVEPKSCDKTHTCPPCPAPELLGGPSVFLFPPKPKDT LMISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLP PSRdeltKNQVSLTCLVKGFYPSDIAVEWESNGQPENNYKTTPPVLDSDGS FFLYSKLTVDKSRWQQGNVFSCSVMHEALHNHYTQKSLSLSPGK<3` |

**Table 17. Light Chain Amino Acid Sequence of Humanized Anti-interleukin 6 Receptor Antibody BAT1806**

| | |
|---|---|
| SEQ ID NO.2 | 5`> <3` |

### Example 12 Expression and Purification of the Humanized Anti-interleukin 6 Receptor Antibody BAT1806

With reference to the method given by Wood et al., J Immunol. 145:3011 (1990*) et al*., the humanized anti-interleukin 6 receptor antibody BAT1806 that specifically binds IL-6R was expressed in CHO cells. The expression vector containing the antibody gene was constructed by a conventional molecular biology method (molecular cloning) using a derived cell line of CHO-kl cells (ATCC CCL61) as a host cell for expression. The construction of a high yield stable cell line is briefly described as follows: host cells were grown in suspension in CD-CHO medium (Gibco, CA), the host cells in logarithmic growth phase were centrifuged, resuspended in fresh CD-CHO medium, the cells were counted and the cell density was adjusted to 1.43x10⁷ cells/mL, 600ul of the above cell suspension was added to an electroporation cuvette, and then 40ug linearized plasmid was added, and pipetting was used to mix the cells with the plasmid uniformly. Electroshock conversion was performed using a Bio-rad electrometer with instrument parameters set as follows: capacitance: 960uFD, and voltage: 300 V. Typically the electric shock are performed for 15-20 milliseconds, which are normal. The electrically shocked cells were immediately resuspended in 37 °C pre-heated CD-CHO medium, inoculated in a 96-well plate at 100ul per well, and 2-3 days later an equal amount of screening medium (CD-CHO media + 50 uM MSX) was added. The 96-well plate cell culture supernatant was analyzed to determine the level of antibody expression. Clones with higher expression levels were transferred from a 96-well plate to a 24-well plate, and after the cells were grown to a certain amount, the cells were transferred to a 6-well plate so that 2x10⁵ cells were contained in 3 mL culture medium per well, and the antibody yield and yield of the cells were measured. Typically 20-30 clones were transferred to shake flasks for further evaluation. The final 5-8 clones with the highest expression were subcloned and further tested for expression. The material liquid was obtained, the cells were separated from the culture medium by low-speed centrifugation, and the supernatant of the centrifugation was further settled by high-speed centrifugation. Affinity purification with protein A and ion exchange purification were performed.

### Example 13 Study on Biological Activity of the Humanized Anti-interleukin-6 Receptor Antibody

BAT1806 antibody Formulations A, B, C and D with an antibody concentration of 20 mg/mL were prepared and tested for cellular activity as described in Example 1. The test method is briefly described as follows: the humanized anti-interleukin 6 receptor antibody BAT1806 standard was used as a standard, the initial concentration was 20ug/mL which was diluted in a gradient manner and added to a 96-well plate, at 50ul per well. The sample to be tested was also diluted according to the standard curve sample dilution method and added to a 96-well plate at 50ul per well. TF-1 cells in logarithmic growth phase were inoculated in a 96-well plate at 100,000 cells/well/100ul, and were incubated for 72 hours in a 37 degree 5% CO₂ incubator. Celltiter Glo reagent was added at 50ul per well. Upon standing for 2 hours in the absence of light at room temperature, microplate data were read via CelltiterGlo method in a microplate reader (Molecular Devices SpectraMax). Four parameters were taken to fit the curve C value (IC50), and according to the equation: the relative specific activity = [IC50 of standard / IC50 of test sample] × 100%, if the calculated results were 80%-125%, the activity of the test sample was considered normal. The results are shown in Table 18.

BAT1806 antibody Formulations A, B, C and D were prepared with an antibody concentration of 20 mg/mL as described in Example 1 and subjected to competitive ELISA test. The test method is briefly described as follows: hIL-6 was diluted to 10 ug/mL in PBS, well mixed and to 100ul per well, i.e., 1ug per well, placed at 4 °C overnight. PBS containing 5% skimmed milk was used to block, 200µl per well, incubated for 2 hours at 37 °C, and the plate was washed with PBST for three times. HIL-6R was diluted to 1 µg/mL, 50µl per well, i.e., 50ng per well, with the dilution solution and placed at room temperature for 30min. BAT1806 was diluted to have an initial concentration of 80µg/mL and then serially diluted to 40, 20, 10, 5, 2.5, 1.25, 0.625, 0.3125, 0.156, 0.08, 0.04µg/mL, and vortexed until evenly. Rabbit anti-his serum was diluted 10,000-fold with dilution solution, and added at 100µl per well, incubated for 1 hour at 37 °C, and the plate was washed for 5 times with PBST. Goat anti-rabbit HRP was subjected to a 10,000-fold dilution, added at 100 µl per well, incubated for 0.5 h at 37 °C, and the plate was washed for 8 times with PBST. TMB chromogenic solution was added at 100µl per well, allowed to stand for 10 minutes at room temperature in the dark, and stopped by addition of 50 µl per well 1 M H₂SO₄. Four-parameter analysis was performed by measuring OD450nm readings with enzyme-labeled detector analysis software SoftMax Pro. Four parameters were taken to fit curve C values (IC50), and according to the equation: the relative specific activity = [standard IC50 / test sample IC50] × 100%, if the calculated results were 80%-125%, the activity of the test sample was considered normal. The results are shown in Table 19.

**Table 18. Cell Activity Test Results for Humanized Anti-interleukin 6 Receptor Antibody**

| Times | Relative activity (%) | | | |
|---|---|---|---|---|
| | Formulation A | Formulation B | Formulation C | Formulation D |
| 1 | 108.5 | 94.5 | 105.3 | 98.5 |
| 2 | 102.4 | 99.3 | 95.7 | 92.8 |
| 3 | 95.7 | 105.2 | 96.5 | 104.3 |
| 4 | 112.2 | 97.1 | 102.4 | 93.2 |
| 5 | 89.9 | 99.2 | 95.9 | 102.6 |
| Mean | 101.7 | 99.1 | 99.2 | 98.3 |
| SD | 9.1 | 4.0 | 4.4 | 5.3 |

**Table 19. Competitive ELISA Test Results for Humanized Anti-interleukin 6 Receptor Antibody**

| Times | Relative activity (%) | | | |
|---|---|---|---|---|
| | Formulation A | Formulation B | Formulation C | Formulation D |
| 1 | 103.5 | 98.4 | 93.7 | 95.8 |
| 2 | 98.2 | 94.1 | 108.2 | 105.7 |
| 3 | 97.4 | 110.5 | 102.4 | 103.9 |
| 4 | 105.1 | 103 | 95.7 | 92.2 |
| 5 | 93.9 | 98.9 | 92 | 95.8 |
| Mean | 99.6 | 101.0 | 98.4 | 98.7 |
| SD | 4.6 | 6.2 | 6.8 | 5.8 |

### Example 14 In vivo Efficacy of the Humanized Anti-interleukin-6 Receptor Antibody

*In vivo* pharmacodynamic evaluation of animals was conducted to investigate the therapeutic effect of the humanized anti-IL-6 receptor antibody on collagen-induced arthritis (CIA) model in cynomolgus monkeys. The specific experimental procedure was as follows: female monkeys were immunized twice with calf type II collagen for CIA modeling. The successfully modeled animals were divided into four groups: negative control NS group, BAT1806 Formulation A group, BAT1806 Formulation B group, BAT1806 Formulation C group, BAT1806 Formulation D group, with 9 monkeys in each group, and were given a single intravenous dose of 30mg/kg. The therapeutic effect of recombinant anti-human interleukin-6 receptor monoclonal antibody on cynomolgus monkey CIA model was evaluated after 4 weeks of continuous observation.

After a single intravenous dose of 30 mg/kg/IV, taking BAT1806 compared with the negative control group, the blood biochemical indexes such as ESR, IL-6, IL-6R and the like of the test animals were significantly improved, and the difference was statistically significant (see Figure 7).

### Example 15 Pharmacokinetics of Humanized Anti-interleukin-6 Receptor Antibody

Pharmacokinetic studies were performed in Wisteria rats. Intravenous injection was performed once with a dose of 12 mg/kg, four groups were set as follows: BAT1806 Formulation A, BAT1806 Formulation B, BAT1806 Formulation C, BAT1806 Formulation D, with 10 rats in each group, female and male in half, blood samples were collected before and immediately after dose, 1h, 2h, 5h, 24h, 48h, 96h, 7 days, 10 days, 14 days, to test the blood concentration; subcutaneous injection was performed once with a dose of 12mg/kg, four groups were set as follows: BAT1806 Formulation A, BAT1806 Formulation B, BAT1806 Formulation C, BAT1806 Formulation D, with 10 rats in each group, female and male in half, and vein blood samples were collected before and immediately after dose, 5h, 8h, 24h, 48h, 96h, 7 days, 10 days, 14 days,. Similar pharmacokinetic profiles were seen for either intravenous or subcutaneous dose in each group, see Figure 8.

### References:

[1] Josef S Smolen, Robert Landewé, Ferdinand C Breedveld, et al. EULAR recommendations for the management of rheumatoid arthritis with synthetic and biological disease-modifying antirheumatic drugs: 2013 update [J]. Ann. Rheum. Dis., 2013, 0:1-18.
[2] Go Woon Kim, et al. IL-6 inhibitors for treatment of rheumatoid arthritis: past, present, and future [J]. Arch. Pharm. Res, 2015, 38:575-584.
[3] Veale DJ, Fearon U. Inhibition of angiogenic pathways in rheumatoid arthritis: potential for therapeutic targeting [J]. Best Pract. Res. Clin. Rheumato., 2006, (20): pp. 941-947.
[4] Xu SD, Tang FI, Shi L, et al. Anti-Saantibody in Chinese Rheumatoid arthritis [J]. Chinese Med. J., 1998, 111(3): 204-207.
[5] Ritchie DM, Boyle JA, Mclnnes JM, et al. Clinical studies with an articular index for the assessment of joint tenderness in patients with rheumatoid arthritis[J]. Q. J. Med, 1986, 37: 393-406.

## Claims

1. An antibody formulation, **characterized by** comprising:
(1) an antibody: 2-100 mg/mL humanized anti-IL-6 receptor antibody;
(2) a buffer;
(3) a surfactant: 0.1-1.0 g/L;
(4) a stabilizer: 30-400 mM;
(5) water for injection;
a pH of the antibody formulation being 5.0-7.0;
wherein a buffer system is formed in the formulation by a buffer, the buffer system being a buffer of 5-20 mM histidine salt, or a combination of 5-20 mM histidine salt and 5-20 mM sodium acetate;
preferably, the concentration of the humanized anti-IL-6 receptor antibody is 10-90 mg/mL; more preferably, the concentration of the humanized anti-IL-6 receptor antibody is 15-50 mg/mL; particularly preferably, the concentration of the humanized anti-IL-6 receptor antibody is 18-25 mg/mL;
preferably, the pH of the antibody formulation is 5.5-6.5; more preferably, the pH of the antibody formulation is 6.0-6.4; still more preferably, the pH of the antibody formulation is 6.2.

2. The antibody formulation according to claim 1, **characterized in that**, the antibody is a recombinant humanized anti-human interleukin 6 receptor monoclonal antibody; preferably, the antibody comprises a heavy chain shown in SEQ ID NO. 1 and a light chain shown in SEQ ID NO. 2; more preferably, the antibody comprises two heavy chains as shown in SEQ ID NO. 1 and two light chains as shown in SEQ ID NO. 2.

3. The antibody formulation according to claim 1 or 2, **characterized in that**, the stabilizer is selected from a combination of arginine hydrochloride and sucrose, or mannitol, or sodium chloride; preferably, the stabilizer is selected from a combination of 40-200 mM arginine hydrochloride and 15-70 g/L sucrose; or preferably, the stabilizer is selected from 30-70 g/L mannitol; or yet preferably, the stabilizer is selected from 100-300 mM sodium chloride;
preferably, the surfactant is selected from one or more of polysorbate-20, polysorbate-80 and poloxamer 188; more preferably, the surfactant is selected from polysorbate-80; more preferably, the surfactant is selected from 0.1-0.7 g/L polysorbate-80.

4. The antibody formulation according to any one of claims 1 to 3, comprising the following ingredients:
(1) 18-22 mg/mL humanized anti-IL-6 receptor antibody;
(2) 8-15 mM histidine salt buffer solution;
(3) 0.45-0.65 g/L polysorbate-80;
(4) 40-60 mM arginine hydrochloride;
(5) 15-25 g/L sucrose;
(6) water for injection;
a pH being 6.0-6.4;
or preferably, comprising the following ingredients:
(1) 18-22 mg/mL humanized anti-IL-6 receptor antibody;
(2) 8-15 mM histidine salt buffer solution;
(3) 0.45-0.65 g/L polysorbate-80;
(4) 30-45 g/L mannitol;
(5) water for injection;
a pH being 6.0-6.4;
or yet preferably, comprising the following ingredients:
(1) 18-22 mg/mL humanized anti-IL-6 receptor antibody;
(2) 8-15 mM histidine salt buffer solution;
(3) 0.45-0.65 g/L polysorbate-80;
(4) 90-110 mM sodium chloride;
(5) water for injection;
a pH being 6.0-6.4.

5. The antibody formulation according to any one of claims 1 to 4, comprising the following ingredients:
(1) 20 mg/mL humanized anti-IL-6 receptor antibody;
(2) 10 mM histidine salt buffer;
(3) 0.5 g/L polysorbate-80;
(4) 50 mM arginine hydrochloride;
(5) 20 g/L sucrose;
(6) water for injection;
a pH being 6.2;
or preferably, comprising the following ingredients:
(1) 20 mg/mL humanized anti-IL-6 receptor antibody;
(2) 10 mM histidine salt buffer;
(3) 0.5 g/L polysorbate-80;
(4) 30 g/L mannitol;
(5) water for injection;
a pH being 6.2;
or preferably, comprising the following ingredients:
(1) 20 mg/mL humanized anti-IL-6 receptor antibody;
(2) 10 mM histidine salt buffer;
(3) 0.5 g/L polysorbate-80;
(4) 42 g/L mannitol;
(5) water for injection;
a pH being 6.2;
or yet preferably, comprising the following ingredients:
(1) 20 mg/mL humanized anti-IL-6 receptor antibody;
(2) 10 mM histidine salt buffer;
(3) 0.5 g/L polysorbate-80;
(4) 100 mM sodium chloride;
(5) water for injection;
a pH being 6.2.

6. The antibody formulation according to any one of claims 1 to 5, **characterized in that**, wherein the formulation also comprises a base; preferably, the base is NaOH.

7. The antibody formulation according to any one of claims 1 to 6, **characterized in that**, the pharmaceutical dosage form of the antibody formulation is an injection formulation; preferably, the formulation is a subcutaneous injection formulation or an intravenous injection formulation.

8. The antibody formulation according to any one of claims 1 to 7, **characterized in that**, the formulation remains stable for at least one month at room temperature; preferably, the formulation remains stable for at least 36 months at 2-8 °C; preferably, the formulation remains stable after at least 5 freeze-thaw cycles.

9. The antibody formulation according to any one of claims 1 to 8, **characterized in that**, wherein the antibody formulation is a pharmaceutical formulation for treating IL-6 related diseases; preferably, the IL-6 related diseases include: adult rheumatoid arthritis, systemic juvenile idiopathic arthritis, polyarticular juvenile idiopathic arthritis, giant cell arteritis, giant lymph node hyperplasia, cytokine storms caused by immunotherapy, adult Still's disease, recurrent polychondritis, type II diabetes, ankylosing spondylitis, thyroid-associated ophthalmopathy, cardiovascular disease caused by rheumatoid arthritis, polymyalgia rheumatica, acute graft-versus-host disease, non-ST-segment elevation myocardial infarction, systemic lupus erythematosus, schizophrenia, uveitis, ovarian cancer, anti-neutrophil cytoplasmic antibody-associated vasculitis, neuromyelitis optica, chronic glomerulonephritis, and colorectal cancer; more preferably, the IL-6 related diseases include: adult rheumatoid arthritis, systemic juvenile idiopathic arthritis, polyarticular juvenile idiopathic arthritis, giant cell arteritis, and giant lymph node hyperplasia.

10. A method to prepare the antibody formulation according to any one of claims 1 to 9, **characterized by** comprising the steps of:
(1) dissolving a weighed buffer, a stabilizer and a surfactant in water for injection;
(2) adjusting the liquid prepared in the step (1) with an aqueous sodium hydroxide until the pH is 5-7; preferably, the concentration of aqueous sodium hydroxide is 1 M;
(3) filtering the liquid prepared in the step (2) into an aseptic container; preferably, the pore size of the filter membrane is 0.22 um; and
(4) adding the liquid prepared in the step (3) into an antibody solution.


SEQUENCE LISTING

<110>  BIO-THERA SOLUTIONS, LTD.

<120>  LIQUID PREPARATION OF HUMANIZED ANTIBODY FOR TREATING IL-6-RELATED
DISEASE

<150>  201711239538.8
<151>  2017-11-30

<160>  2

<170>  SIPOSequenceListing 1.0

<210>  1
<211>  449
<212>  PRT
<213>  Artificial Sequence

<220>
<223> Heavy Chain Amino Acid Sequence of Humanized Anti-interleukin 6
Receptor Antibody BAT1806

<400>  1
Gln Val Gln Leu Gln Glu Ser Gly Pro Gly Leu Val Arg Pro Ser Gln
1               5                   10                  15
Thr Leu Ser Leu Thr Cys Thr Val Ser Gly Tyr Ser Ile Thr Ser Asp
            20                  25                  30
His Ala Trp Ser Trp Val Arg Gln Pro Pro Gly Arg Gly Leu Glu Trp
        35                  40                  45
Ile Gly Tyr Ile Ser Tyr Ser Gly Ile Thr Thr Tyr Asn Pro Ser Leu
    50                  55                  60
Lys Ser Arg Val Thr Met Leu Arg Asp Thr Ser Lys Asn Gln Phe Ser
65                  70                  75                  80
Leu Arg Leu Ser Ser Val Thr Ala Ala Asp Thr Ala Val Tyr Tyr Cys
                85                  90                  95
Ala Arg Ser Leu Ala Arg Thr Thr Ala Met Asp Tyr Trp Gly Gln Gly
            100                 105                 110
Ser Leu Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Lys
    210                 215                 220
Thr His Thr Cys Pro Pro Cys Pro Ala Pro Glu Leu Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu

305                 310                 315                 320
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Asp Glu Leu Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys


<210>  2
<211>  214
<212>  PRT
<213>  Artificial Sequence

<220>
<223> Light Chain Amino Acid Sequence of Humanized Anti-interleukin 6
Receptor Antibody BAT1806

<400>  2